# EUROPEAN PATENT APPLICATION

(11) **EP 4 810 058 A1**
(43) Date of publication of application: **23.09.2026**
(21) Application number: 24890795.8
(22) Date of filing: 15.11.2024
(51) Int. Cl.: A61K 45/06, A61K 39/395, A61K 31/513, A61K 31/7068, A61K 33/243, A61P 35/00

(54) **METHOD FOR TREATING GASTRIC CANCER BY COMBINING ANTI-HER2 ANTIBODY AND CHEMOTHERAPEUTIC AGENT, AND USE**

(30) Priority: 16.11.2023 CN 202311527171
(71) Applicant: Shanghai Henlius Biotech, Inc., Shanghai 201210 (CN); Shanghai Henlius Biologics Co., Ltd., Shanghai 201616 (CN); Shanghai Henlius Biopharmaceutical Co., Ltd., Shanghai 200233 (CN)
(72) Inventor: WANG, Qingyu, Shanghai 201210 (CN); YANG, Futang, Shanghai 201210 (CN); CHENG, Jiancheng, Shanghai 201210 (CN); QIU, Wenting, Shanghai 201210 (CN)
(74) Representative: Rentsch Partner AG
(86) International application number: PCT/CN2024/132197
(87) International publication number: WO 2025/103441

(57) **Abstract**

Trastuzumab, a second anti-HER2 antibody and a chemotherapeutic agent are combined to treat HER2-positive advanced gastric cancer. Compared with existing first-line treatment solutions for HER2-positive advanced gastric cancer, the combined treatment method of trastuzumab, the second anti-HER2 antibody and the chemotherapeutic agent (XELOX) prolongs the progression free survival (PFS) and/or the overall survival (OS) of patients.

## Description

### CROSS-REFERENCE TO RELATED APPLICATION

The present application claims priority to Chinese Patent Application No. CN202311527171.5 filed on Nov. 16, 2023, which is incorporated by reference in its entirety.

### TECHNICAL FIELD

The present disclosure relates to a method for treating cancer with a pharmaceutical combination and use thereof. In particular, the present disclosure relates to a method for treating HER2-positive gastric cancer or gastroesophageal junction adenocarcinoma with a combination of an anti-HER2 antibody, trastuzumab, and a chemotherapeutic agent and use thereof.

### BACKGROUND

Gastric cancer is a common malignancy. According to WHO statistics, gastric cancer ranked fourth among malignant tumor deaths worldwide with 754,000 deaths in 2015. In China, gastric cancer is the second most common malignancy reported annually and the most common malignant neoplasm in the digestive tract in China, with a high incidence worldwide. Gastric cancer can be classified into adenocarcinoma, adenosquamous cell carcinoma, squamous cell carcinoma, carcinoid, etc., and the vast majority of gastric cancers are gastric adenocarcinomas. The clinical early stage of gastric cancer is insidious. Although the rapid development of gastrointestinal endoscopy has greatly improved the early diagnosis rate of gastric cancer, the prognosis of gastric cancer is generally still unsatisfactory, especially the prognosis of locally advanced and metastatic gastric cancers. Although surgical resection can provide the greatest treatment opportunity for gastric cancer patients, postoperative recurrence and metastasis are also important causes of death of such gastric cancer patients.

The HER2 (human epidermal growth factor receptor 2) gene is an oncogene located on the long arm of human chromosome 17 (17q21-q22) and encodes a transmembrane glycoprotein receptor. HER2 is a receptor tyrosine kinase that binds to the surface of the cell membrane. It is involved in the signaling pathway leading to cell growth and differentiation, and is encoded by the protooncogene HER2/neu. The scientific community generally considers HER2 as an orphan receptor. None of the ligands of the epidermal growth factor family can activate HER2. However, when the ligand binds to an ErbB receptor, a dimer can be formed, and HER2 can bind to other members of the ErbB (tyrosine kinase receptor) family to form a heterodimer.

HER2-positive gastric cancer is a unique disease subtype, which requires diagnosis and treatment strategies and methods different from those for HER2-negative gastric cancer. The positive rate of HER2 overexpression in gastric cancer reported worldwide is 7.3% to 20.2%, while the positive rate of HER2 in Chinese patients with gastric cancer is 12% to 13%. At present, the value of HER2 in the prognosis of gastric cancer lacks consensus due to different evaluation criteria used in different studies.

Patients with HER2-positive advanced gastric cancer can benefit from the treatment with trastuzumab. Previously, based on existing nonclinical results and the confirmatory clinical benefits of trastuzumab in combination with chemotherapy for HER2-positive metastatic breast cancer and adjuvant therapy of breast cancer, a randomized phase III study (ToGA) was conducted to evaluate the efficacy and safety of the combination of a fluorouracil drug (capecitabine or 5-FU) + cisplatin (FP) with trastuzumab in the treatment of HER2-positive advanced gastric cancer. The study enrolled 594 patients in 24 countries. The patients were randomized in a 1:1 ratio to receive the FP regimen or FP + trastuzumab (TFP) treatment. The primary endpoint was OS. The results show that the TFP treatment was superior to the FP monotherapy, and in the Intention-to-Treat (ITT) population, the median overall survival of the patients in the TFP group was 13.8 months, compared with 11.1 months for the FP treatment group (risk ratio = 0.74; 95% CI: 0.60, 0.91). The median progression-free survival was 6.7 months for patients in the TFP group and 5.5 months for the FP treatment group (risk ratio = 0.71; 95% CI: 0.59, 0.85).

For the chemotherapeutic agent options for patients with advanced gastric cancer, a two-drug combination regimen of fluorouracil and platinum-based drugs is generally recommended in China. Based on the good tolerability of patients and the actual clinical application in China, oxaliplatin is more recommended among platinum-based drugs in the CSCO guidelines for gastric cancer. The efficacy of the capecitabine + oxaliplatin (XELOX) regimen has also been confirmed by related clinical trials, and has been recommended as level I by the CSCO guidelines for gastric cancer in China. Meanwhile, the current European ESMO guidelines and the NCCN guidelines in the United States also recommend trastuzumab in combination with chemotherapy (fluorouracil and platinum-based drugs) as the first-line treatment for HER2-positive advanced gastric cancer. Immunotherapy has become an important research direction for the combination treatment of HER2-positive advanced gastric cancer.

### SUMMARY

A first objective of the present disclosure is to provide use of trastuzumab in combination with a second antibody and a chemotherapeutic agent in preparing a medicament for treating HER2-positive gastric cancer.

A second objective of the present disclosure is to provide a kit for use in treating HER2-positive gastric cancer or gastroesophageal junction adenocarcinoma.

The present disclosure further provides a method for treating HER2-positive gastric cancer or gastroesophageal junction adenocarcinoma with a combination of trastuzumab, a second antibody, and a chemotherapeutic agent.

According to one aspect of the present disclosure, the present disclosure provides use of a combination of trastuzumab, a second antibody, and a chemotherapeutic agent in treating cancer, particularly HER2-positive gastric cancer, wherein the second antibody is an anti-HER2 antibody (also referred to as a second anti-HER2 antibody) or an antigen-binding fragment thereof, and the second antibody has a different binding epitope from trastuzumab; preferably, the second antibody comprises a heavy chain and a light chain:
the heavy chain comprises a heavy chain variable region comprising the HCDR1 (heavy chain complementarity determining region 1) set forth in SEQ ID NO: 1, the HCDR2 (heavy chain complementarity determining region 2) set forth in SEQ ID NO: 2, and the HCDR3 (heavy chain complementarity determining region 3) set forth in SEQ ID NO: 3; and
the light chain comprises a light chain variable region comprising the LCDR1 (light chain complementarity determining region 1) set forth in SEQ ID NO: 6, the LCDR2 (light chain complementarity determining region 2) set forth in SEQ ID NO: 7, and the LCDR3 (light chain complementarity determining region 3) set forth in SEQ ID NO: 8.

In one preferred embodiment of the present disclosure, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In one preferred embodiment of the present disclosure, the heavy chain of the second antibody comprises or consists of the amino acid sequence set forth in SEQ ID NO: 5, and the light chain of the second antibody comprises or consists of the amino acid sequence set forth in SEQ ID NO: 10.

As the chemotherapeutic agent for use in combination with the two antibodies, various chemotherapeutic agents currently used in clinics or recommended by guidelines can be selected, and the preferred chemotherapeutic agents are fluorouracil drugs and platinum-based drugs used alone or in combination. Preferably, the fluorouracil drug is selected from 5-fluorouracil and capecitabine, and the platinum-based drug is selected from cisplatin and oxaliplatin. More preferably, the chemotherapeutic agent is capecitabine and oxaliplatin, and in the most preferred treatment regimen, trastuzumab is used in combination with a second antibody and a capecitabine + oxaliplatin chemotherapy regimen (XELOX).

The use described herein is mainly for treating HER2-positive gastric cancer, particularly HER2-positive advanced gastric cancer, and more preferably advanced unresectable or metastatic HER2-positive gastric cancer, including gastroesophageal junction adenocarcinoma, which generally has the same or similar diagnostic criteria and clinical manifestations as gastric cancer. In one preferred embodiment of the present disclosure, the method and use of the present disclosure are for previously untreated patients diagnosed with advanced unresectable or metastatic gastric cancer, i.e., for the first-line treatment of the cancer.

In terms of the mode of administration and dosage, the dosages of trastuzumab and the second antibody or the binding fragment thereof are as follows: trastuzumab is administered intravenously once every 3 weeks, with an initial loading dose of 8 mg/kg followed by doses of 6 mg/kg once every 3 weeks, and the second antibody is administered at a dose of 25 mg/kg or 15 mg/kg; the chemotherapeutic agent is the combination of capecitabine with oxaliplatin (the XELOX regimen), in which oxaliplatin is administered intravenously at a dose of 130 mg/m² once every three weeks, and capecitabine is administered orally at a dose of 1000 mg/m² twice daily in cycles of three weeks.

On the basis of the above combination therapy, an immune checkpoint inhibitor, such as an anti-PD-1 antibody and/or an anti-PD-L1 antibody, preferably an anti-PD-1 antibody, may also be administered as desired.

According to another aspect of the present disclosure, in the combination therapy of the present disclosure, the drugs may be administered to a patient separately in accordance with the above regimens or may be prepared in the form of a kit for ease of administration. Therefore, the present disclosure further provides a kit for use in treating HER2-positive gastric cancer or gastroesophageal junction adenocarcinoma, comprising:
trastuzumab;
a second antibody, wherein the second antibody is a second anti-HER2 antibody or an antigen-binding fragment thereof having a different binding site from trastuzumab, comprising a heavy chain and a light chain:
   the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
   a chemotherapeutic agent.

In one preferred embodiment of the present disclosure, the chemotherapeutic agent is capecitabine and oxaliplatin.

The kit described herein may further comprise an anti-PD-1 antibody and/or an anti-PD-L1 antibody.

### Beneficial Effects:

The present disclosure provides a method and use of an anti-HER2 antibody combination in combination with a chemotherapeutic agent for treating HER2-positive gastric cancer in a patient. In particular, the method and use are for treating HER2-positive advanced gastric cancer with the combination of trastuzumab, a second anti-HER2 antibody, and a chemotherapeutic agent. The data from a randomized, double-blind, multi-site, phase II clinical study of a combination therapy of trastuzumab, a second anti-HER2 antibody, and a chemotherapeutic agent (XELOX) in patients with advanced unresectable or metastatic HER2-positive gastric cancer suggest that compared to the first-line treatment regimen of HER2-positive advanced gastric cancer, the combination therapy of trastuzumab, the second anti-HER2 antibody, and the chemotherapeutic agent (XELOX) prolongs the progression-free survival (PFS) and/or the overall survival (OS) of the patients.

### BRIEF DESCRIPTION OF THE DRAWINGS

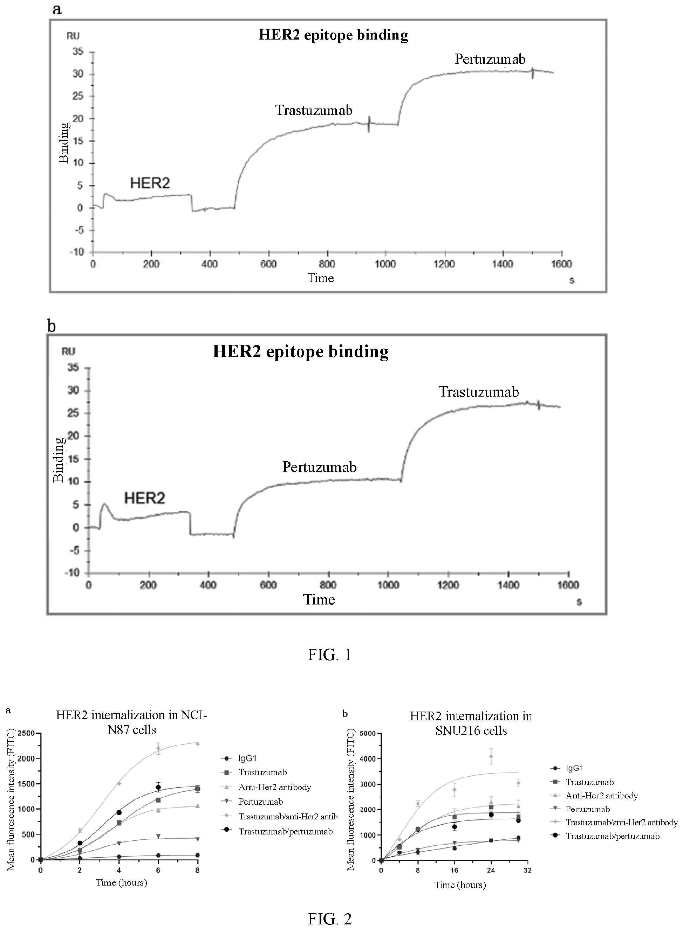

FIG. 1 shows the results of a HER2 epitope binding competition assay of the second anti-HER2 antibody, trastuzumab, and pertuzumab according to the present disclosure,
   where a shows the results of the epitope binding competition assay in which the HER2-Fc protein, trastuzumab, and pertuzumab were injected sequentially;
   b shows the results of the epitope binding competition assay in which the HER2-Fc protein, pertuzumab, and trastuzumab were injected sequentially;
   the HER2 in the figure denotes the HER2-Fc protein.
FIG. 2 shows a study on HER2-mediated endocytosis in gastric cancer cells NCI-N87 (a) and SNU216 (b) treated with a combination of a second anti-HER2 antibody and trastuzumab according to the present disclosure.
FIG. 3 shows a study on the EGFR cell expression level caused by treating gastric cancer NCI-N87 cells with the combination of a second anti-HER2 antibody and trastuzumab according to the present disclosure,
   where a, b, and c show the expression levels of HER2 in NCI-N87 cells at different time points;
   where d, e, and f show the expression levels of EGFR in NCI-N87 cells at different time points.
FIG. 4a shows a study on the cell viability of gastric cancer NCI-N87 cells treated with the combination of a second anti-HER2 antibody and trastuzumab according to the present disclosure;
FIG. 4b shows a study on the apoptosis of gastric cancer NCI-N87 cells treated with the combination of a second anti-HER2 antibody and trastuzumab according to the present disclosure;
FIG. 4c shows the results of tumor volume in a xenograft human gastric cancer NCI-N87 model treated with the combination of a second anti-HER2 antibody and trastuzumab according to the present disclosure;
FIGs. 4d and 4e show the results of tumor volume of a human gastric cancer PDX model treated with the combination of a second anti-HER2 antibody and trastuzumab according to the present disclosure.
FIG. 5 shows the progression-free survival Kaplan-Meier curves (RECIST1.1) of subjects in a clinical study according to the present disclosure.

### DETAILED DESCRIPTION

The present disclosure will be further described in detail with reference to the following embodiments, which should not be construed as limiting the present disclosure.

### I. Definitions

The term "HER receptor" refers to receptor protein tyrosine kinases of the HER receptor family, including EGFR, HER2, HER3, and HER4 receptors. The HER receptor generally comprises: a. an extracellular domain that can bind to a HER ligand and/or dimerize with another HER receptor molecule; b. a lipophilic transmembrane domain; c. a conserved intracellular tyrosine kinase domain; and d. a carboxyl-terminal signaling domain containing several tyrosine residues that can be phosphorylated. The HER receptor may be a "native sequence" HER receptor or an "amino acid sequence variant" thereof. Preferably, the HER receptor is a native sequence human HER receptor.

"HER dimer" herein refers to a non-covalently associated dimer comprising at least two HER receptors. Such complexes may form when cells expressing two or more HER receptors are exposed to HER ligands. These complexes can be isolated by immunoprecipitation and analyzed by SDS-PAGE, as described in, for example, Sliwkowski et al., J. Biol. Chem. 269(20):14661-14665 (1994). Other proteins, such as cytokine receptor subunits, can associate with the dimer. Preferably, the HER dimer comprises HER2.

"HER heterodimer" herein refers to a non-covalently associated heterodimer comprising at least two different HER receptors, such as an EGFR-HER2, HER2-HER3, or HER2-HER4 heterodimer. "HER antibody" refers to an antibody that binds to a HER receptor. Optionally, the HER antibody further interferes with HER activation or function. Preferably, the HER antibody binds to a HER2 receptor. The HER2 antibody involved herein is trastuzumab and a second anti-HER2 antibody, which comprise CDRs.

"HER activation" refers to the activation or phosphorylation of any one or more HER receptors. Generally, HER activation results in signal transduction (e.g., phosphorylation of tyrosine residues in HER receptors or substrate polypeptides, which is caused by the intracellular kinase domain of HER receptors). HER activation may be mediated by a HER ligand that binds to a HER dimer comprising a HER receptor. A HER ligand that binds to a HER dimer may activate the kinase domain of one or more HER receptors in the dimer and thereby cause phosphorylation of tyrosine residues in one or more HER receptors and/or phosphorylation of tyrosine residues in other substrate polypeptides such as the intracellular kinases Akt or MAPK.

"Phosphorylation" refers to the addition of one or more phosphate groups to a protein, such as a HER receptor, or a substrate thereof.

An antibody that "inhibits HER dimerization" refers to an antibody that inhibits or interferes with the formation of a HER dimer. Preferably, such antibodies bind to HER2 at the heterodimerization binding site thereof. The most preferred dimerization-inhibiting antibody herein is trastuzumab or a second anti-HER2 antibody. Other examples of antibodies that inhibit HER dimerization include antibodies that bind to EGFR and inhibit dimerization thereof with one or more other HER receptors; antibodies that bind to HER3 and inhibit dimerization thereof with one or more other HER receptors; and antibodies that bind to HER4 and inhibit dimerization thereof with one or more other HER receptors.

"HER2 dimerization inhibitor" is an agent that inhibits the formation of a dimer or a heterodimer comprising HER2.

The "heterodimerization binding site" on HER2 refers to a region in the extracellular domain of HER2 that contacts a region in the extracellular domain of EGFR, HER3, or HER4 or forms an interface with a region in the extracellular domain of EGFR, HER3, or HER4 when forming a dimer with EGFR, HER3, or HER4. This region has been found in domain II of HER2.

As used herein, "trastuzumab" refers to an antibody comprising a heavy chain of SEQ ID NO: 11 and a light chain of SEQ ID NO: 12, and CDRs contained therein. It can be understood that the term "trastuzumab" herein encompasses "rhuMb4D5" (e.g., the antibody disclosed in U.S. Patent No. 5,821,337) and biosimilars of trastuzumab drugs.

"Pertuzumab" refers to an antibody comprising a heavy chain of SEQ ID NO: 13 and a light chain of SEQ ID NO: 14, and CDRs contained therein. It can be understood that the term "pertuzumab" herein encompasses "RhuMAb 2C4" (e.g., as disclosed in WO01/00245, WO2006/007398, and U.S. Pat. No. US2006/0034842) and biosimilars of pertuzumab drugs.

The term "antibody" is used herein in the broadest sense and specifically encompasses monoclonal antibodies, polyclonal antibodies, multispecific antibodies, and antibody fragments. In some embodiments, the term "antibody" refers to a protein comprising at least two heavy (H) chains and two light (L) chains interconnected by disulfide bonds. Each heavy chain consists of a heavy chain variable region (abbreviated herein as VH) and a heavy chain constant region (abbreviated herein as CH). In certain antibodies, such as naturally occurring IgG antibodies, the heavy chain constant region comprises a hinge and three domains, i.e., CH1, CH2, and CH3. In some antibodies, such as naturally occurring IgG antibodies, each light chain comprises a light chain variable region (abbreviated herein as VL) and a light chain constant region. The light chain constant region consists of one domain (abbreviated herein as CL). The VH and VL regions may be further subdivided into hypervariable regions, called complementarity determining regions (CDRs), which are interspersed with more conserved regions called framework regions (FRs). Each VH and VL consists of three CDRs and four FRs arranged from the amino terminus to the carboxyl terminus in the following order: FR1, CDR1, FR2, CDR2, FR3, CDR3, and FR4. The variable regions of the heavy and light chains comprise binding domains that interact with antigens. The constant regions of the antibody can mediate the binding of immunoglobulins to host tissues or factors, including various cells of the immune system (e.g., effector cells) and the first component (C1q) of the classical complement system. The heavy chain may or may not have a C-terminal lysine. Unless otherwise stated herein, the amino acids in the variable regions are numbered using the Kabat scheme, while the amino acids in the constant regions are numbered using the EU scheme.

"Intact antibody" herein refers to an antibody comprising two antigen-binding regions and an Fc region.

"Antigen-binding fragment" herein refers to any antibody fragment capable of binding to an antigen, including Fab, F(ab'), F(ab')2, Fv, etc. Fab has one antigen-binding site, which is composed of a heavy chain variable domain and a light chain variable domain, a light chain constant domain, and a first heavy chain constant domain (CH1) of an antibody. Fab' differs from Fab by having a hinge region comprising one or more cysteine residues at the C-terminus of the heavy chain CH1 domain. An F(ab')2 antibody is produced by forming disulfide bonds between cysteine residues in the hinge regions of Fab' fragments. Fv is the smallest antibody fragment composed of a heavy chain variable region and a light chain variable region, and recombinant techniques for preparing Fv fragments are disclosed in PCT WO 88/10649, WO 88/106630, WO 88/07085, WO 88/07086, and WO 88/09344. In a double-chain construct, the variable regions of the heavy and light chains are linked by non-covalent bonds, whereas in a single-chain Fv, the variable regions of the heavy and light chains are generally linked by covalent bonds via a peptide linker or are directly linked to each other at the C-terminus to form a dimer (e.g., a dsFv-like structure). Such antibody fragments can be obtained using proteolytic enzymes (e.g., papain digestion of a whole antibody produces Fab fragments, while treatment with pepsin results in the production of F(ab')₂ fragments), and can be prepared by genetic recombinant techniques.

The term "heavy chain" used herein refers to both a full-length heavy chain and a portion thereof, which comprises a variable domain (VH) containing an amino acid sequence of a variable region sequence for specifically binding to an antigen, and three constant domains (CH1, CH2, and CH3).

The term "light chain" used herein refers to both a full-length light chain and a portion thereof, which comprises a variable domain (VL) containing an amino acid sequence of a variable region sequence for specifically binding to an antigen, and a constant domain (CL).

The term "CDR (complementarity determining region)" used herein refers to amino acid sequences of the heavy and light chain hypervariable regions of an immunoglobulin (Kabat et al., Sequences of Proteins of Immunological Interest, 4th Ed., U.S. Department of Health and Human Services, National Institutes of Health (1987)). Each heavy chain and light chain comprises three CDRs (heavy chain (HCDR1, HCDR2, and HCDR3) and light chain (LCDR1, LCDR2, and LCDR3)). CDRs provide contact residues that have an important role in the binding of an antibody to an antigen or epitope.

"Epitope" or "binding epitope", also known as an antigenic determinant, refers to a site on an antigen molecule that is capable of specifically binding to an antibody-binding site and determines the specificity of the antigen.

As used herein, "immune checkpoint" is a class of immunosuppressive molecules that are mainly expressed on immune cells and can regulate the degree of immune activation and prevent autoimmunity; examples include PD1, CTLA4, LMTK3, LAG3, TIM3, TIGIT, etc. "Immune checkpoint inhibitor" refers to an agent that is capable of reducing, blocking, inhibiting, eliminating, or interfering with the interaction of an immune checkpoint molecule with a ligand thereof. In some embodiments, the immune checkpoint inhibitor includes an anti-PD-1 antibody, an anti-CTLA4 antibody, an anti-LMTK3 antibody, an anti-LAG3 antibody, an anti-TIM3 antibody, and an anti-TIGIT antibody. In certain specific embodiments of the present disclosure, the immune checkpoint inhibitor is preferably an anti-PD-1 antibody.

"PD-1", programmed death receptor 1, refers to an immunosuppressive receptor that binds to the CD28 family. PD-1 is mainly expressed on the surface of activated T cells *in vivo* and is capable of binding to two ligands, PD-L1 and PD-L2. The term "PD-1" generally includes human PD-1, variants, isotypes, or analogs having at least one universal epitope thereof. As used herein, the anti-PD-1 antibody is capable of reducing, blocking, inhibiting, eliminating, or interfering with signal transduction of the interaction between PD-1 and PD-L1 and/or PD-L2.

As used herein, "ADCC or antibody-dependent cell-mediated cytotoxicity" refers to the effect that after an IgG antibody specifically binds to a surface antigenic determinant of a target cell (e.g., a tumor cell or an infected cell) via an Fab fragment, the Fc fragment of the antibody binds to an effector cell with FcγRs, e.g., an NK cell, a monocyte-macrophage, a neutrophil, etc., triggering the killing activity of the effector cell to directly kill the target cell, wherein the NK cell is a primary cell mediating ADCC.

"Cell line-derived xenograft tumor model" or "CDX" refers to a tumor model constructed by transplanting a heterologous (e.g., human) tumor cell line cultured *in vitro* into immunodeficient mice. The tumor model is currently one of the most common *in vivo* models for preclinical pharmacodynamic evaluation of anti-tumor drugs.

"PDX model" or "patient-derived xenograft model" refers to a model where a tumor tissue from a tumor patient is transplanted into severely immunodeficient mice, and the tumor tissue is allowed to grow in the mice to form an xenograft tumor. The model can retain the growth microenvironment of the parent tumor, facilitates a better representation of the characteristics of the parent tumor, and maintains the heterogeneity of the tumor.

As used herein, "cancer" or "carcinoma" refers to a physiological disorder in mammals that is characterized by uncontrolled cellular hyperproliferation. As used herein, "cancer" refers to a large group of diseases characterized by the uncontrolled growth of abnormal cells in the body. "Cancer" or "cancerous tissue" may include a tumor. Uncontrolled cell division may lead to the formation of malignant tumors or cells that invade adjacent tissues and may metastasize to distant parts of the body through the lymphatic system or the bloodstream. After metastasis, a distant tumor can be said to be "derived from" a pre-metastasis tumor. For example, a tumor "derived from" melanoma refers to a tumor resulting from metastatic melanoma. Since the distal tumor is derived from a pre-metastasis tumor, the tumor "derived from" a pre-metastasis tumor may also include the pre-metastasis tumor. For example, a tumor derived from melanoma may include melanoma. In some embodiments, the cancer or tumor includes a solid tumor. In some embodiments, the cancer or tumor includes an advanced solid tumor. In some embodiments, the cancer or tumor includes a solid tumor that has spread. In some embodiments, the cancer or tumor includes an advanced malignant tumor. In some embodiments, the cancer or tumor is a metastatic cancer or tumor (e.g., a stage 4 cancer or tumor).

As used herein, "advanced gastric cancer" refers to gastric cancer that has spread beyond the original site or organ due to local invasion or metastasis. Generally, advanced gastric cancer is determined according to the AJCC/UICC TNM staging system for gastric cancer, e.g., tumor invasion to tissues (e.g., visceral peritoneum or adjacent structures), regional lymph node metastasis and/or distant metastasis, or signs of CT staging.

"Unresectable" cancer refers to cancer that cannot be surgically resected, primarily due to tumor-related reasons, e.g., severe invasion of the primary tumor into surrounding tissues, making the cancer indistinguishable from adjacent normal tissues or encasing major blood vessels; fixed regional lymph node metastasis with confluent mass formation, or metastatic lymph nodes located beyond surgically accessible areas; distant metastasis or peritoneal carcinomatosis, etc.

"Metastatic" cancer refers to cancer that spreads from one part of the body (generally the primary site) to another part of the body.

"HER2-positive" cancer refers to cancer that contains cancer cells with a higher-than-normal level of HER2. Generally, HER2-positive cancer has an immunohistochemistry (IHC) score of 2+ or 3+ and/or an *in situ* hybridization (ISH) amplification ratio of ≥ 2.0.

The term "patient" refers to a human subject receiving prophylactic or therapeutic treatment.

As used herein, the term "subject" refers to a human. In some embodiments, the subject has not been subjected to treatment. In some embodiments, the subject has received at least one prior therapy for treating a cancer or tumor. In some embodiments, the subject has received, and then progressed on, relapsed from, or developed intolerance to at least one standard treatment regimen. There are a variety of standard of care therapies known in the art for particular types of cancers or tumors. In some embodiments, at least one standard treatment regimen includes treatment in an advanced or metastatic setting according to solid tumor histology.

"Treatment" refers to therapeutic treatment and prophylactic or preventative measures, taken to obtain beneficial or desired outcomes, including clinical outcomes. Obtaining beneficial or desired outcomes includes, but is not limited to, one or more of the following: alleviating one or more symptoms caused by a disease, reducing the severity of the disease, stabilizing the disease (e.g., preventing or delaying disease worsening), delaying or slowing the progression of the disease, improving the state of the disease, increasing or improving quality of life, increasing body weight, and/or extending survival.

"Administer", "administering", or "administration" refers to introducing a therapeutic drug to an entity (e.g., a subject) using one of a variety of methods and delivery modes or systems known to those skilled in the art. The route of administration of the antibody includes intravenous, intramuscular, subcutaneous, intraperitoneal, and other parenteral routes. In some embodiments, the antibody is administered intravenously, e.g., by injection or infusion. The route of administration of the chemotherapeutic agent includes intravenous, intramuscular, subcutaneous, and other parenteral routes of administration, or oral administration.

As used herein, the term "about once every week", "about once every two weeks", "about once every three weeks", or any other similar dosage interval terms refers to an approximation. "About once every week" may include once every 7 ± 1 days, i.e., once every 6 days to once every 8 days. "About once every two weeks" may include once every fourteen ± three days, i.e., once every eleven days to once every seventeen days. "About once every three weeks" may include once every twenty-one days ± three days, i.e., once every eighteen days to once every twenty-four days. For example, similar approximations apply to about once every four weeks, about once every five weeks, about once every six weeks, and about once every twelve weeks. In some embodiments, a dosage interval of about once every six weeks or about once every twelve weeks means that the first dose can be administered on any day of the first week and then the next dose can be administered on any day of the sixth or twelfth week. In some embodiments, a dosage interval of about once every six weeks or about once every twelve weeks means that the first dose is administered on a particular day (e.g., Monday) of the first week and then the next dose is administered on the same day (i.e., Monday) of the sixth or twelfth week.

As used herein, "effective dose or effective amount" refers to an amount that is capable of producing a therapeutic effect in an individual upon each administration. This dose may vary depending on a variety of factors, such as the treatment objective, the treatment frequency, the body weight and tolerance of the individual, the severity of the symptoms, the risk of side effects, and the route of administration. Specifically, the term "effective amount" or "effective dose" is defined as an amount sufficient to achieve, or at least partially achieve, the desired effect. A "therapeutically effective amount" or "therapeutically effective dose" of a drug or therapeutic agent refers to any amount of the drug that, when administered alone or in combination with another therapeutic agent, can promote disease regression as evidenced by a reduction in the severity of disease symptoms, an increase in the frequency and duration of asymptomatic periods, or the prevention of damage or disability due to the disease affliction. The therapeutically effective amount or dose of the drug includes a "prophylactically effective amount" or "prophylactically effective dose", which is any amount of the drug that, when administered alone or in combination with another therapeutic agent to a subject at risk of developing a disease or experiencing disease recurrence, inhibits the occurrence or recurrence of the disease. The ability of a therapeutic agent to promote disease regression or inhibit the occurrence or recurrence of a disease can be assessed using a variety of methods known to those skilled in the art, e.g., in a human subject during a clinical trial, in an animal model system for predicting the efficacy of a therapeutic agent in a human, or by determining the activity of an agent in an *in vitro* assay.

For example, an anti-cancer agent is a drug that promotes the regression of cancer in a subject. In some embodiments, the therapeutically effective amount of the drug promotes the regression of the cancer to an extent that eliminates the cancer. "Promoting cancer regression" means that the administration of an effective amount of the drug, alone or in combination with an anti-tumor drug, results in a decrease in tumor growth or size, tumor necrosis, a reduction in the severity of at least one disease symptom, an increase in the frequency and duration of the asymptomatic period, prevention of damage or disability due to the disease affliction, or otherwise the alleviation of the disease symptoms in the patient. In addition, the terms "effective" and "effectiveness" with respect to treatment include pharmaceutical efficacy and physiological safety. Pharmaceutical efficacy refers to the ability of a drug to promote cancer regression in a patient. Physiological safety refers to the level of toxicity or other adverse physiological effects (adverse effects) at the cellular, organ and/or organism level resulting from the administration of a drug.

As an example of treating a tumor, a therapeutically effective amount or dose of the drug inhibits cell growth or tumor growth by at least about 20%, at least about 40%, at least about 60%, or at least about 80% relative to a subject who has not been subjected to treatment. In some embodiments, the therapeutically effective amount or dose of the drug completely inhibits cell growth or tumor growth, i.e., inhibits cell growth or tumor growth by 100%. The ability of a compound to inhibit tumor growth can be assessed using the assays described below. Alternatively, such properties of a composition can be assessed by examining the ability of the compound to inhibit cell growth, and such inhibition can be measured *in vitro* by assays known to those skilled. As used herein, the term "body weight-based" amount or dose refers to a dose administered to a patient that is calculated on the basis of the weight of the patient. For example, when a patient with a body weight of 60 kg requires 15 mg/kg of the second antibody, an appropriate amount of the second antibody (i.e., 900 mg) can be calculated and administered.

"Chemotherapeutic agent" refers to a chemical compound that can be used to treat cancer. For example, chemotherapeutic agents used in chemotherapy include alkylating agents, ethylenimines and methylamelamines, aceogenins, colchicines, camptothecins, antibiotics, folic acid analogs, purine analogs, pyrimidine analogs, platinum analogs, platinum-based analogs, etc. The chemotherapeutic agent used in the present disclosure relates to a fluorouracil compound and a platinum-based chemotherapeutic agent, specifically including 5-fluorouracil (5-FU), capecitabine, cisplatin, and oxaliplatin. In addition, "XELOX" used in the present disclosure refers to a combination chemotherapeutic agent comprising drugs capecitabine (Xeloda) and oxaliplatin, which is generally used in the chemotherapy of gastrointestinal cancers, such as advanced gastric cancer and advanced colon cancer.

As used herein, "chemotherapy" is used to refer to any chemotherapeutic method for treating cancer, including standard regimens of chemotherapy with a fluorouracil compound and chemotherapy with a platinum-based chemotherapeutic agent involved in the present disclosure. In specific embodiments of the present disclosure, the chemotherapeutic agent includes 5-fluorouracil, capecitabine + cisplatin or oxaliplatin, preferably capecitabine + oxaliplatin.

"Immunotherapy" refers to the treatment of an entity with a disease or at risk of infection or disease recurrence by a method that includes inducing, enhancing, suppressing, or otherwise altering an immune response.

As used herein, a "fixed" or "flat" dose refers to a dose that is suitable for a patient without considering the body weight and body surface area of the patient.

A "loading" dose generally includes an initial dose of a therapeutic agent (including trastuzumab, a second anti-HER2 antibody, a chemotherapeutic agent, etc.) administered to a patient, followed by one or more maintenance doses thereof. In general, a single loading dose is loaded; while in other embodiments of the present disclosure, multiple loading doses are loaded. Generally, the amount of the loading dose administered exceeds the amount of the maintenance dose administered, and/or the loading dose is administered more frequently than the maintenance dose, such that the desired steady-state concentration of the therapeutic agent is achieved earlier than with the maintenance dose.

A "maintenance" dose herein refers to one or more doses of a therapeutic agent administered to a patient during treatment. Generally, the maintenance dose is administered at certain treatment intervals, e.g., about once every week, about once every two weeks, about once every three weeks, or about once every four weeks, preferably once every three weeks.

"Intravenous (IV)" administration refers to administration of a drug (e.g., trastuzumab, a second anti-HER2 antibody, or a chemotherapeutic agent) into the vein of a patient, e.g., by infusion. "Subcutaneous" administration refers to administration of a drug (including trastuzumab, a second anti-HER2 antibody, or a chemotherapeutic agent) under the skin of a patient.

As used herein, "overall survival" or "OS" refers to the survival of a patient for a defined period of time, such as 1 year and 5 years, from the time of self-diagnosis or treatment. For the purpose of the clinical trial described in the examples, overall survival (OS) is defined as the time from the date of randomization of the patient population to the date of death from any cause.

"Progression-free survival" or "PFS" means that the patient remains alive without progression or worsening of the cancer. Progression-free survival (PFS) is defined as the time from randomization of the study population to first documented progressive disease or unmanageable toxicity or death from any cause, whichever occurs first. Disease progression can be documented by any clinically accepted method.

"Disease-free survival" or "DFS" refers to a period of time during which a patient remains alive without recurrence of the cancer from start of treatment or from initial diagnosis, such as about 1 year, about 2 years, about 3 years, about 4 years, about 5 years, and about 10 years. In the studies that form the basis of the present disclosure, DFS is analyzed in accordance with the intention-to-treat principle, i.e., patients are evaluated on the basis of the therapy to which they are assigned. Events used in DFS analysis typically include local, regional and distant cancer recurrence, secondary cancer occurrence, and death from any cause in patients without a prior event (gastric cancer recurrence or second primary cancer).

"Invasive disease-free survival" or "iDFS" is the time after adjuvant treatment during which a patient remains alive without recurrence of invasive cancer at any site or death from any cause. In other words, iDFS is defined as a period of time during which a patient remains alive (survival) without recurrence of the invasive disease from start of treatment or from initial diagnosis after adjuvant treatment, such as about 1 year, about 2 years, about 3 years, about 4 years, about 5 years, and about 10 years. In one embodiment, iDFS is about 1 year or about 3 years from start of treatment.

"Prolonged survival" means prolonging overall or progression-free survival in a patient treated in accordance with the present disclosure relative to an untreated patient and/or relative to a patient treated with one or more approved anti-tumor agents but not receiving treatment in accordance with the present disclosure. In one specific example, "prolonged survival" means prolonging progression-free survival (PFS) and/or overall survival of a cancer patient receiving the combination therapy of the present disclosure relative to a patient treated with trastuzumab and chemotherapy alone. In another specific example, "prolonged survival" means prolonging progression-free survival (PFS) and/or overall survival (OS) of a cancer patient receiving the combination therapy of the present disclosure relative to a patient treated with the antibody and/or the chemotherapeutic agent alone.

"Objective response" refers to a measurable response, including complete response (CR) or partial response (PR).

"Complete response" or "CR" means that all signs of cancer disappear in response to treatment. This does not always mean that the cancer is cured.

"Partial response" or "PR" refers to the reduction in size of one or more tumors or lesions, or the extent of cancer in the body in response to treatment.

As used herein, an "adverse event (AE)" is any adverse and often unintended or undesirable sign (including abnormal laboratory findings), symptom, or disease associated with the use of medical treatment. For example, an adverse event may be associated with activation of the immune system or expansion of immune system cells in response to the treatment.

As used herein, "safety data" relates to data obtained in a controlled clinical trial showing the incidence and severity of adverse events to guide users on the safety of the drug, including guidance on how to monitor and prevent adverse reactions to the drug.

"Efficacy data" refers to data obtained in a controlled clinical trial showing that a drug is effective in treating a disease, e.g., gastric cancer.

As used herein, "concurrent" use refers to administration on the same day of treatment with the one or more additional drugs and optionally at the same time as the one or more additional drugs, during the same treatment cycle.

As used herein, "combined use" or "use in combination" means that two or more active substances are administered to a patient or subject as a mixture, simultaneously as a single formulation, or sequentially in any order as a single formulation.

The term "synergistic effect" or "synergy" means that the effect produced by two or more active molecules is greater than the simple addition of the effects of ingredients administered separately. The term "about" is used to mean approximately, substantially, roughly, or in a range of.... The term "about", when used in conjunction with a numerical range, adjusts the range by extending the boundaries above and below the stated numerical value. Generally, the term "about" can adjust a numerical value above or below the stated value with a variance above or below (higher or lower), e.g., 10%.

### II. Overview

The present disclosure provides use of an anti-HER2 antibody in combination with a chemotherapeutic agent in preparing a medicament for treating gastric cancer, specifically use of a combination of trastuzumab, a second anti-HER2 antibody or an antigen-binding fragment thereof, and a chemotherapeutic agent in preparing a medicament for treating HER2-positive gastric cancer, particularly HER2-positive advanced gastric cancer, and more preferably use in treating a patient diagnosed with advanced unresectable or metastatic HER2-positive gastric cancer or gastroesophageal junction adenocarcinoma.

The present disclosure further provides a method for treating the diseases described above, in particular for treating a patient diagnosed with advanced unresectable or metastatic HER2-positive gastric cancer or gastroesophageal junction adenocarcinoma, which comprises administering to the patient a therapeutically effective amount of trastuzumab, a second anti-HER2 antibody or a binding fragment thereof, and a chemotherapeutic agent (e.g., a fluorouracil chemotherapeutic agent and a platinum-based compound).

In some embodiments of the present disclosure, the treatment prolongs the progression-free survival (PFS) and/or overall survival (OS) of the patient, and the condition of the patient is significantly improved according to the objective response rate (ORR).

### III. Anti-HER2 antibody and chemotherapeutic agent

The anti-HER2 antibody involved in the present disclosure includes trastuzumab and a second anti-HER2 antibody, the mechanisms of action of which include inhibiting PI3K/AKT signaling pathway, inducing cell cycle arrest, mediating antibody-dependent cell-mediated cytotoxicity (ADCC), inhibiting DNA damage repair, inhibiting angiogenesis, inducing immune response, etc.

### Trastuzumab

Trastuzumab includes Herceptin (trastuzumab) and a biosimilar thereof, wherein the heavy chain of the monoclonal antibody comprises or consists of the amino acid sequence of SEQ ID NO: 11, and the light chain comprises or consists of the amino acid sequence of SEQ ID NO: 12. Trastuzumab is used for treating HER2-positive metastatic breast cancer, and HER2-positive early breast cancer and metastatic gastric cancer (according to a package insert approved by the U.S. Food and Drug Administration, and a package insert approved by the National Medical Product Administration of China). In an applicable dosing regimen for HER2-positive metastatic breast cancer, trastuzumab is used as a single drug for treating metastatic breast cancer in a patient who has received one or more chemotherapy regimens, and is used in combination with paclitaxel or docetaxel for a patient with metastatic breast cancer who has not received chemotherapy, with an initial loading dose of 4 mg/kg administered via intravenous infusion (IV) over 90 min and a maintenance dose of 2 mg/kg. In an applicable dosing regimen for metastatic gastric cancer involved in the present disclosure, trastuzumab is administered once every three weeks with an initial loading dose of 8 mg/kg followed by 6 mg/kg once every three weeks, wherein the patient with metastatic gastric cancer receives treatment with trastuzumab until disease progression or intolerable toxicity.

### Second antibody (also referred to as second anti-HER2 antibody)

The second antibody (also referred to as second anti-HER2 antibody) involved in the present disclosure is a monoclonal antibody that binds to the HER2 site and is different from trastuzumab. In some embodiments, the second anti-HER2 antibody comprises a heavy chain variable region and a light chain variable region, wherein:
(a) the heavy chain variable region comprises the HCDR1 of SEQ ID NO: 1, the HCDR2 of SEQ ID NO: 2, and the HCDR3 of SEQ ID NO: 3;
(b) the light chain variable region comprises the LCDR1 of SEQ ID NO: 6, the LCDR2 of SEQ ID NO: 7, and the LCDR3 of SEQ ID NO: 8.

In some embodiments, the second anti-HER2 antibody comprises a heavy chain variable region and a light chain variable region, wherein:
(a) the heavy chain variable region comprises or consists of the amino acid sequence of SEQ ID NO: 4;
(b) the light chain variable region comprises or consists of the amino acid sequence of SEQ ID NO: 9.

In some embodiments, the second anti-HER2 antibody comprises a heavy chain and a light chain, wherein:
(a) the heavy chain comprises or consists of the amino acid sequence of SEQ ID NO: 5;
(b) the light chain comprises or consists of the amino acid sequence of SEQ ID NO: 10.

Other embodiments of the present disclosure relate to the use of an antigen-binding fragment of a second anti-HER2 antibody, which comprises the amino acid sequences defined above, particularly antigen-binding structural regions such as HCDR1, HCDR2, HCDR3, LCDR1, LCDR2 and LCDR3, examples of which include Fab, Fab', F(ab')₂ and Fv fragments, diabodies, linear antibodies, single-chain antibody molecules, and multispecific antibodies formed from other antibody fragments.

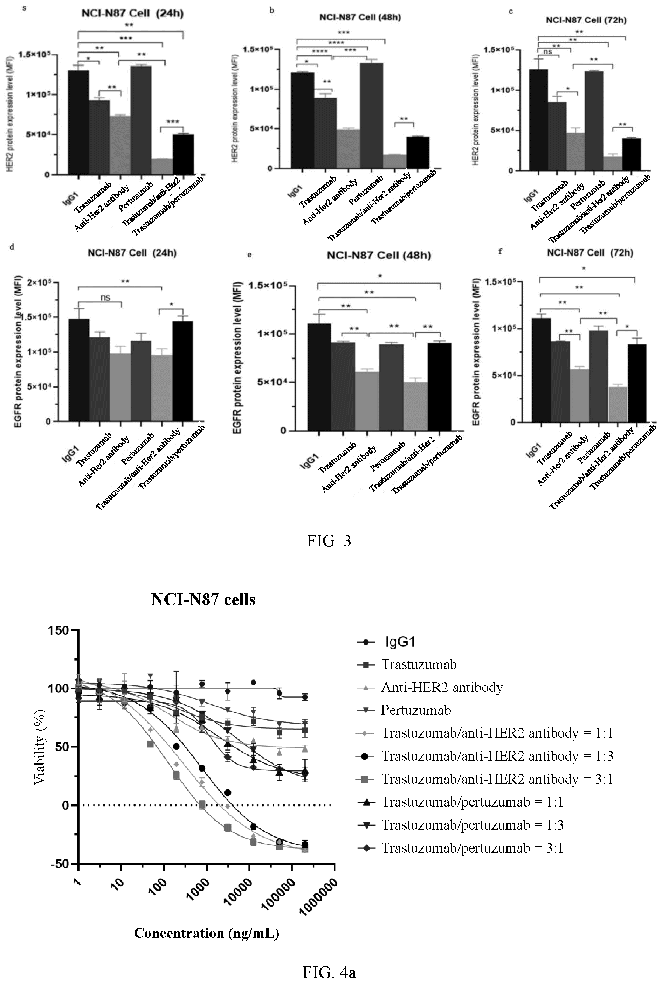

The second anti-HER2 antibody used in the present disclosure is a humanized IgG1 monoclonal antibody, and has the same HER2-targeting domain, i.e., domain IV of HER2, as trastuzumab. However, trastuzumab and the second anti-HER2 antibody act on different epitopes, and the second anti-HER2 antibody does not compete with trastuzumab for binding to domain IV of HER2. See FIG. 1 for the analysis of HER2 epitope binding of the second anti-HER2 antibody, pertuzumab, and trastuzumab. Endocytosis is one of the mechanisms by which antibodies are used as tumor therapeutic drugs. The antibody-receptor interaction or complex can promote efficient endocytosis of receptor molecules in cells, so that the receptors on the cell surface are reduced and degraded, inhibiting receptor-dependent signaling and affecting the growth of tumor cells. Regarding the second anti-HER2 antibody used in the present disclosure and trastuzumab, the applicant has found that the second anti-HER2 antibody and trastuzumab, when acting alone on HER2-positive gastric cancer cells, induce weak endocytosis of HER2 on the surface of gastric cancer cells, thereby inducing weak effects on inducing apoptosis and inhibiting tumor cell growth. As shown in FIG. 2, the second anti-HER2 antibody, trastuzumab, and pertuzumab alone induce weak endocytosis in gastric cancer cells (NCI-N87 and SNU216). However, the combination of the second anti-HER2 antibody and trastuzumab induces significantly enhanced endocytosis in the gastric cancer cells, whereas the combination of trastuzumab and pertuzumab does not induce enhanced endocytosis in the gastric cancer cells. Referring to FIG. 3, the endocytosis in gastric cancer cells induced by the combination of the second anti-HER2 antibody and trastuzumab results in a decrease in the expression levels of HER2 and EGFR in gastric cancer cells, whereas the combination of trastuzumab and pertuzumab results in a decrease in the HER2 expression level, but a smaller decrease in the EGFR level, in gastric cancer cells. Therefore, it can be reasonably presumed that although the combination of trastuzumab and pertuzumab can be used for adjuvant therapy of HER2-positive breast cancer (e.g., as described in CN110337450, Roche), the combination of trastuzumab and pertuzumab may be difficult to be used for the treatment or adjuvant therapy of HER2-positive gastric cancer due to the limitations of different tumor cell characteristics and antibody mechanisms. Correspondingly, according to the studies in the examples of the present disclosure, the combination of trastuzumab and the second anti-HER2 antibody can be used for a novel therapeutic regimen for HER2-positive gastric cancer.

In some embodiments, the present disclosure relates to a composition comprising trastuzumab and a second anti-HER2 antibody or a binding fragment thereof, and a pharmaceutically acceptable carrier, wherein the second anti-HER2 antibody is as defined above.

### Fluorouracil compound and platinum-based chemotherapeutic agent

### Fluorouracil compound

Fluorouracil compounds are among the most widely used anti-tumor drugs clinically. They kill cancer cells by preventing the formation of pyrimidine nucleotides and interfering with DNA synthesis, and include 5-FU (5-fluorouracil) and oral prodrugs such as capecitabine and tegafur. Clinically, they are mainly used for treating breast cancer, gastrointestinal cancer, ovarian cancer, and primary bronchopulmonary adenocarcinoma.

Some embodiments of the present disclosure relate to use of 5-FU or capecitabine. In a more preferred embodiment, capecitabine is used for the use and method involved in the present disclosure.

### Platinum-based chemotherapeutic agent

Platinum-based chemotherapeutic agents are also widely used anti-tumor drugs. They can induce DNA cross-linking to form monoadducts, interstrand cross-links, intrastrand cross-links, or DNA-protein cross-links, which act on the adjacent N-7 positions of guanine to form 1,2-intrastrand cross-links (Poklar et al. (1996). Proc. Natl. Acad. Sci. U.S.A. 93(15): 7606-7611; Rudd et al.

(1995). Cancer Chemother. Pharmacol. 35(4): 323-326); the resulting cross-links inhibit DNA repair and/or DNA synthesis in cancer cells. Platinum-based chemotherapeutic agents include cisplatin, carboplatin, oxaliplatin, and staraplatin.

Some embodiments of the present disclosure relate to use of cisplatin or oxaliplatin. In a more preferred embodiment, oxaliplatin is used for the use and method involved in the present disclosure.

### IV. Treatment method and use

The present disclosure relates to use of a combination of trastuzumab, a second anti-HER2 antibody or a binding fragment thereof, and a chemotherapeutic agent in preparing a medicament for treating HER2-positive gastric cancer, particularly for treating a patient diagnosed with advanced unresectable or metastatic HER2-positive gastric cancer or gastroesophageal junction adenocarcinoma.

In another aspect, the present disclosure further relates to a method for treating HER2-positive gastric cancer, particularly for treating a patient diagnosed with advanced unresectable or metastatic HER2-positive gastric cancer or gastroesophageal junction adenocarcinoma, which comprises administering to the patient a therapeutically effective amount of trastuzumab, a second anti-HER2 antibody or a binding fragment thereof, and a chemotherapeutic agent. In some embodiments, the treatment of the present disclosure prolongs the progression-free survival (PFS) and/or overall survival (OS) of the patient, and the condition of the patient is significantly improved according to the objective response rate (ORR).

The present disclosure specifically relates to a treatment regimen for advanced gastric cancer, particularly advanced unresectable or metastatic HER2-positive gastric cancer or gastroesophageal junction adenocarcinoma. The advanced gastric cancer involved in the present disclosure can be determined according to the AJCC/UICC TNM rule, for example, according to the progression of tumor invasion to surrounding tissues, the status of regional lymph node metastasis or distant metastasis, and signs of CT staging. HER2 detection is applied to patients with advanced gastric cancer treated in the present disclosure. For example, HER2 positivity is defined in the KEYNOT811 study, i.e., being based on immunohistochemistry (IHC) 3+ or immunohistochemistry (IHC) 2+ positivity with *in situ* hybridization (ISH) positivity. In some embodiments of the present disclosure, HER2-positive cancer is defined based on the expression level of HER2 corresponding to immunohistochemistry (IHC) 3+ or 2+ with ISH positivity. The immunohistochemistry (IHC) and *in situ* hybridization (ISH) assays for HER2 are all performed in full accordance with the operating specifications of The Guidelines for HER2 Detection in Gastric Cancer. For the relevant IHC and ISH assays, commercially available assays approved by the FDA or the National Medical Products Administration (NMPA) of China may be selected.

In some embodiments of the present disclosure, the use or method comprises a combination of trastuzumab, a second anti-HER2 antibody or a binding fragment thereof, and a chemotherapeutic agent. In some embodiments, the second anti-HER2 antibody or the binding fragment thereof is as defined above. The chemotherapeutic agent is a combination of a fluorouracil compound and a platinum-based chemotherapeutic agent. In some specific embodiments, the fluorouracil compound is 5-fluorouracil or capecitabine. In some specific embodiments, the platinum-based compound is cisplatin or oxaliplatin. In some more preferred embodiments, the chemotherapeutic agent is a combination of 5-fluorouracil and oxaliplatin.

Specifically, in one aspect, the present disclosure provides a method for treating HER2-positive gastric cancer or gastroesophageal junction adenocarcinoma in a patient, which comprises administering to the patient a therapeutically effective amount of:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

In a further embodiment, the chemotherapeutic agent is a fluorouracil drug. In a further embodiment, the chemotherapeutic agent is a platinum-based drug.

In a further embodiment, the chemotherapeutic agent is a combination of a fluorouracil drug and a platinum-based drug.

In a further embodiment, the fluorouracil drug is selected from 5-fluorouracil and capecitabine.

In a further embodiment, the platinum-based drug is selected from cisplatin and oxaliplatin. In a further embodiment, the fluorouracil drug is capecitabine. In a further embodiment, the platinum-based drug is oxaliplatin. In a further embodiment, the chemotherapeutic agent is a combination of capecitabine and oxaliplatin.

In a further embodiment, the cancer is HER2-positive advanced gastric cancer.

In a further embodiment, the cancer is unresectable or metastatic HER2-positive gastric cancer or gastroesophageal junction adenocarcinoma.

In a further embodiment, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In a further embodiment, the heavy chain of the second antibody comprises or consists of the amino acid sequence set forth in SEQ ID NO: 5, and the light chain of the second antibody comprises or consists of the amino acid sequence set forth in SEQ ID NO: 10.

In a further embodiment, trastuzumab is administered at a body weight-based dose of about 0.1 mg/kg to about 10 mg/kg.

In a further embodiment, trastuzumab is administered at a body weight-based dose of about 0.1 mg/kg to about 10 mg/kg, about 0.3 mg/kg to about 10 mg/kg, 0.9 mg/kg to about 10 mg/kg, about 1 mg/kg to about 10 mg/kg, about 2.5 mg/kg to about 10 mg/kg, about 3 mg/kg to about 10 mg/kg, about 4 mg/kg to about 10 mg/kg, about 5 mg/kg to about 10 mg/kg, about 6 mg/kg to about 10 mg/kg, about 7 mg/kg to about 10 mg/kg, about 8 mg/kg to about 10 mg/kg, about 9 mg/kg to about 10 mg/kg, about 0.1 mg/kg to about 8 mg/kg, about 0.3 mg/kg to about 8 mg/kg, 0.9 mg/kg to about 8 mg/kg, about 1 mg/kg to about 8 mg/kg, about 2.5 mg/kg to about 8 mg/kg, about 3 mg/kg to about 8 mg/kg, about 4 mg/kg to about 8 mg/kg, about 5 mg/kg to about 8 mg/kg, about 6 mg/kg to about 8 mg/kg, about 7 mg/kg to about 8 mg/kg, about 0.1 mg/kg to about 6 mg/kg, about 0.3 mg/kg to about 6 mg/kg, 0.9 mg/kg to about 6 mg/kg, about 1 mg/kg to about 6 mg/kg, about 2.5 mg/kg to about 6 mg/kg, about 3 mg/kg to about 6 mg/kg, about 4 mg/kg to about 6 mg/kg, or about 5 mg/kg to about 6 mg/kg.

In a further embodiment, trastuzumab is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

In a further embodiment, on a body weight basis, trastuzumab is administered at an initial loading dose of about 8 mg/kg followed by a dose of about 6 mg/kg.

In a further embodiment, trastuzumab is administered about once every week, about once every two weeks, about once every three weeks, about once every four weeks, about once every month, about once every 3-6 months, or longer.

In a further embodiment, trastuzumab is administered intravenously.

In a further embodiment, the second antibody is administered at a body weight-based dose of about 0.1 mg/kg to about 30 mg/kg, about 0.3 mg/kg to about 30 mg/kg, 0.9 mg/kg to about 30 mg/kg, about 1 mg/kg to about 30 mg/kg, about 2.5 mg/kg to about 30 mg/kg, about 3 mg/kg to about 30 mg/kg, about 4 mg/kg to about 30 mg/kg, about 5 mg/kg to about 30 mg/kg, about 6 mg/kg to about 30 mg/kg, about 7 mg/kg to about 30 mg/kg, about 8 mg/kg to about 30 mg/kg, about 9 mg/kg to about 30 mg/kg, about 10 mg/kg to about 30 mg/kg, about 11 mg/kg to about 30 mg/kg, about 12 mg/kg to about 30 mg/kg, about 13 mg/kg to about 30 mg/kg, about 14 mg/kg to about 30 mg/kg, about 15 mg/kg to about 30 mg/kg, about 16 mg/kg to about 30 mg/kg, about 17 mg/kg to about 30 mg/kg, about 18 mg/kg to about 30 mg/kg, about 19 mg/kg to about 30 mg/kg, about 20 mg/kg to about 30 mg/kg, about 21 mg/kg to about 30 mg/kg, about 22 mg/kg to about 30 mg/kg, about 23 mg/kg to about 30 mg/kg, about 24 mg/kg to about 30 mg/kg, about 25 mg/kg to about 30 mg/kg, about 26 mg/kg to about 30 mg/kg, about 27 mg/kg to about 30 mg/kg, about 28 mg/kg to about 30 mg/kg, about 29 mg/kg to about 30 mg/kg, about 0.1 mg/kg to about 25 mg/kg, about 0.3 mg/kg to about 25 mg/kg, 0.9 mg/kg to about 25 mg/kg, about 1 mg/kg to about 25 mg/kg, about 2.5 mg/kg to about 25 mg/kg, about 3 mg/kg to about 25 mg/kg, about 4 mg/kg to about 25 mg/kg, about 5 mg/kg to about 25 mg/kg, about 6 mg/kg to about 25 mg/kg, about 7 mg/kg to about 25 mg/kg, about 8 mg/kg to about 25 mg/kg, about 9 mg/kg to about 25 mg/kg, about 10 mg/kg to about 25 mg/kg, about 11 mg/kg to about 25 mg/kg, about 12 mg/kg to about 25 mg/kg, about 13 mg/kg to about 25 mg/kg, about 14 mg/kg to about 25 mg/kg, about 15 mg/kg to about 25 mg/kg, about 16 mg/kg to about 25 mg/kg, about 17 mg/kg to about 25 mg/kg, about 18 mg/kg to about 25 mg/kg, about 19 mg/kg to about 25 mg/kg, about 20 mg/kg to about 25 mg/kg, about 21 mg/kg to about 25 mg/kg, about 22 mg/kg to about 25 mg/kg, about 23 mg/kg to about 25 mg/kg, about 24 mg/kg to about 25 mg/kg, about 0.1 mg/kg to about 20 mg/kg, about 0.3 mg/kg to about 20 mg/kg, 0.9 mg/kg to about 20 mg/kg, about 1 mg/kg to about 20 mg/kg, about 2.5 mg/kg to about 20 mg/kg, about 3 mg/kg to about 20 mg/kg, about 4 mg/kg to about 20 mg/kg, about 5 mg/kg to about 20 mg/kg, about 6 mg/kg to about 20 mg/kg, about 7 mg/kg to about 20 mg/kg, about 8 mg/kg to about 20 mg/kg, about 9 mg/kg to about 20 mg/kg, about 10 mg/kg to about 20 mg/kg, about 11 mg/kg to about 20 mg/kg, about 12 mg/kg to about 20 mg/kg, about 13 mg/kg to about 20 mg/kg, about 14 mg/kg to about 20 mg/kg, about 15 mg/kg to about 20 mg/kg, about 16 mg/kg to about 20 mg/kg, about 17 mg/kg to about 20 mg/kg, about 18 mg/kg to about 20 mg/kg, about 19 mg/kg to about 20 mg/kg, about 0.1 mg/kg to about 15 mg/kg, about 0.3 mg/kg to about 15 mg/kg, 0.9 mg/kg to about 15 mg/kg, about 1 mg/kg to about 15 mg/kg, about 2.5 mg/kg to about 15 mg/kg, about 3 mg/kg to about 15 mg/kg, about 4 mg/kg to about 15 mg/kg, about 5 mg/kg to about 15 mg/kg, about 6 mg/kg to about 15 mg/kg, about 7 mg/kg to about 15 mg/kg, about 8 mg/kg to about 15 mg/kg, about 9 mg/kg to about 15 mg/kg, about 10 mg/kg to about 15 mg/kg, about 11 mg/kg to about 15 mg/kg, about 12 mg/kg to about 15 mg/kg, about 13 mg/kg to about 15 mg/kg, or about 14 mg/kg to about 15 mg/kg.

In a further embodiment, the second antibody is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, or about 30 mg/kg.

In a further embodiment, the second antibody is administered at a body weight-based dose of about 25 mg/kg or 15 mg/kg.

In a further embodiment, the second antibody is administered about once every week, about once every two weeks, about once every three weeks, about once every four weeks, about once every month, about once every 3-6 months, or longer.

In a further embodiment, the second antibody is administered intravenously.

In a further embodiment, oxaliplatin is administered at a body surface area-based dose of about 10 to about 150 mg/m², about 20 to about 150 mg/m², about 30 to about 150 mg/m², about 40 to about 150 mg/m², about 50 to about 150 mg/m², about 60 to about 150 mg/m², about 70 to about 150 mg/m², about 75 to about 150 mg/m², about 80 to about 150 mg/m², about 85 to about 150 mg/m², about 90 to about 150 mg/m², about 95 to about 150 mg/m², about 100 to about 150 mg/m², about 105 to about 150 mg/m², about 110 to about 150 mg/m², about 115 to about 150 mg/m², about 120 to about 150 mg/m², about 125 to about 150 mg/m², about 130 to about 150 mg/m², about 135 to about 150 mg/m², about 140 to about 150 mg/m², about 145 to about 150 mg/m², about 10 to about 140 mg/m², about 20 to about 140 mg/m², about 30 to about 140 mg/m², about 40 to about 140 mg/m², about 50 to about 140 mg/m², about 60 to about 140 mg/m², about 70 to about 140 mg/m², about 75 to about 140 mg/m², about 80 to about 140 mg/m², about 85 to about 140 mg/m², about 90 to about 140 mg/m², about 95 to about 140 mg/m², about 100 to about 140 mg/m², about 105 to about 140 mg/m², about 110 to about 140 mg/m², about 115 to about 140 mg/m², about 120 to about 140 mg/m², about 125 to about 140 mg/m², about 130 to about 140 mg/m², about 135 to about 140 mg/m², about 10 to about 130 mg/m², about 20 to about 130 mg/m², about 30 to about 130 mg/m², about 40 to about 130 mg/m², about 50 to about 130 mg/m², about 60 to about 130 mg/m², about 70 to about 130 mg/m², about 75 to about 130 mg/m², about 80 to about 130 mg/m², about 85 to about 130 mg/m², about 90 to about 130 mg/m², about 95 to about 130 mg/m², about 100 to about 130 mg/m², about 105 to about 130 mg/m², about 110 to about 130 mg/m², about 115 to about 130 mg/m², about 120 to about 130 mg/m², or about 125 to about 130 mg/m².

In a further embodiment, oxaliplatin is administered at a body surface area-based dose of about 10 mg/m², about 20 mg/m², about 30 mg/m², about 40 mg/m², about 50 mg/m², about 60 mg/m², about 70 mg/m², about 75 mg/m², about 80 mg/m², about 85 mg/m², about 90 mg/m², about 95 mg/m², about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², or about 150 mg/m².

In a further embodiment, oxaliplatin is administered at a body surface area-based dose of about 130 mg/m².

In a further embodiment, oxaliplatin is administered about once every week, about once every two weeks, about once every three weeks, about once every four weeks, about once every month, about once every 3-6 months, or longer.

In a further embodiment, oxaliplatin is administered intravenously.

In a further embodiment, capecitabine is administered at a body surface area-based dose of about 100 to about 1200 mg/m², about 200 to about 1200 mg/m², about 300 to about 1200 mg/m², about 400 to about 1200 mg/m², about 500 to about 1200 mg/m², about 600 to about 1200 mg/m², about 650 to about 1200 mg/m², about 700 to about 1200 mg/m², about 750 to about 1200 mg/m², about 800 to about 1200 mg/m², about 850 to about 1200 mg/m², about 900 to about 1200 mg/m², about 950 to about 1200 mg/m², about 1000 to about 1200 mg/m², about 1050 to about 1200 mg/m², about 1100 to about 1200 mg/m², about 1150 to about 1200 mg/m², about 100 to about 1100 mg/m², about 200 to about 1100 mg/m², about 300 to about 1100 mg/m², about 400 to about 1100 mg/m², about 500 to about 1100 mg/m², about 600 to about 1100 mg/m², about 650 to about 1100 mg/m², about 700 to about 1100 mg/m², about 750 to about 1100 mg/m², about 800 to about 1100 mg/m², about 850 to about 1100 mg/m², about 900 to about 1100 mg/m², about 950 to about 1100 mg/m², about 1000 to about 1100 mg/m², about 100 to about 1000 mg/m², about 200 to about 1000 mg/m², about 300 to about 1000 mg/m², about 400 to about 1000 mg/m², about 500 to about 1000 mg/m², about 600 to about 1000 mg/m², about 650 to about 1000 mg/m², about 700 to about 1000 mg/m², about 750 to about 1000 mg/m², about 800 to about 1000 mg/m², about 850 to about 1000 mg/m², about 900 to about 1000 mg/m², or about 950 to about 1000 mg/m².

In a further embodiment, capecitabine is administered at a body surface area-based dose of about 100 mg/m², about 200 mg/m², about 300 mg/m², about 400 mg/m², about 500 mg/m², about 600 mg/m², about 650 mg/m², about 700 mg/m², about 750 mg/m², about 800 mg/m², about 850 mg/m², about 900 mg/m², about 950 mg/m², about 1000 mg/m², about 1050 mg/m², about 1100 mg/m², about 1150 mg/m², or about 1200 mg/m².

In a further embodiment, capecitabine is administered at a body surface area-based dose of about 1000 mg/m².

In a further embodiment, capecitabine is administered once daily, twice daily, or thrice daily.

In a further embodiment, capecitabine is administered orally.

In a further embodiment, on a body weight basis, trastuzumab is administered at an initial loading dose of about 8 mg/kg followed by a dose of about 6 mg/kg; the second antibody is administered at a body weight-based dose of about 25 mg/kg or 15 mg/kg; oxaliplatin is administered at a body surface area-based dose of about 130 mg/m²; capecitabine is administered at a body surface area-based dose of about 1000 mg/m².

In a further embodiment, the second antibody is administered to the patient before the administration of trastuzumab.

In a further embodiment, the second antibody is administered to the patient after the administration of trastuzumab.

In a further embodiment, trastuzumab and the second antibody are administered concurrently.

In a further embodiment, the method further comprises administering an immune checkpoint inhibitor.

In a further embodiment, the immune checkpoint inhibitor is an anti-PD-1 antibody or an anti-PD-L1 antibody.

In a further embodiment, the immune checkpoint inhibitor is an anti-PD-1 antibody.

In another aspect, the present disclosure further provides a combination therapy for treating HER2-positive gastric cancer or gastroesophageal junction adenocarcinoma in a patient, and the treatment comprises administering to the patient the combination therapy, wherein the combination therapy comprises:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

In a further embodiment, the chemotherapeutic agent is a fluorouracil drug. In a further embodiment, the chemotherapeutic agent is a platinum-based drug.

In a further embodiment, the chemotherapeutic agent is a combination of a fluorouracil drug and a platinum-based drug.

In a further embodiment, the fluorouracil drug is selected from 5-fluorouracil and capecitabine.

In a further embodiment, the platinum-based drug is selected from cisplatin and oxaliplatin. In a further embodiment, the fluorouracil drug is capecitabine. In a further embodiment, the platinum-based drug is oxaliplatin. In a further embodiment, the chemotherapeutic agent is a combination of capecitabine and oxaliplatin.

In a further embodiment, the cancer is HER2-positive advanced gastric cancer.

In a further embodiment, the cancer is unresectable or metastatic HER2-positive gastric cancer or gastroesophageal junction adenocarcinoma.

In a further embodiment, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In a further embodiment, the heavy chain of the second antibody comprises or consists of the amino acid sequence set forth in SEQ ID NO: 5, and the light chain of the second antibody comprises or consists of the amino acid sequence set forth in SEQ ID NO: 10.

In a further embodiment, trastuzumab is administered at a body weight-based dose of about 0.1 mg/kg to about 10 mg/kg.

In a further embodiment, trastuzumab is administered at a body weight-based dose of about 0.1 mg/kg to about 10 mg/kg, about 0.3 mg/kg to about 10 mg/kg, 0.9 mg/kg to about 10 mg/kg, about 1 mg/kg to about 10 mg/kg, about 2.5 mg/kg to about 10 mg/kg, about 3 mg/kg to about 10 mg/kg, about 4 mg/kg to about 10 mg/kg, about 5 mg/kg to about 10 mg/kg, about 6 mg/kg to about 10 mg/kg, about 7 mg/kg to about 10 mg/kg, about 8 mg/kg to about 10 mg/kg, about 9 mg/kg to about 10 mg/kg, about 0.1 mg/kg to about 8 mg/kg, about 0.3 mg/kg to about 8 mg/kg, 0.9 mg/kg to about 8 mg/kg, about 1 mg/kg to about 8 mg/kg, about 2.5 mg/kg to about 8 mg/kg, about 3 mg/kg to about 8 mg/kg, about 4 mg/kg to about 8 mg/kg, about 5 mg/kg to about 8 mg/kg, about 6 mg/kg to about 8 mg/kg, about 7 mg/kg to about 8 mg/kg, about 0.1 mg/kg to about 6 mg/kg, about 0.3 mg/kg to about 6 mg/kg, 0.9 mg/kg to about 6 mg/kg, about 1 mg/kg to about 6 mg/kg, about 2.5 mg/kg to about 6 mg/kg, about 3 mg/kg to about 6 mg/kg, about 4 mg/kg to about 6 mg/kg, or about 5 mg/kg to about 6 mg/kg.

In a further embodiment, trastuzumab is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

In a further embodiment, on a body weight basis, trastuzumab is administered at an initial loading dose of about 8 mg/kg followed by a dose of about 6 mg/kg.

In a further embodiment, trastuzumab is administered about once every week, about once every two weeks, about once every three weeks, about once every four weeks, about once every month, about once every 3-6 months, or longer.

In a further embodiment, trastuzumab is administered intravenously.

In a further embodiment, the second antibody is administered at a body weight-based dose of about 0.1 mg/kg to about 30 mg/kg, about 0.3 mg/kg to about 30 mg/kg, 0.9 mg/kg to about 30 mg/kg, about 1 mg/kg to about 30 mg/kg, about 2.5 mg/kg to about 30 mg/kg, about 3 mg/kg to about 30 mg/kg, about 4 mg/kg to about 30 mg/kg, about 5 mg/kg to about 30 mg/kg, about 6 mg/kg to about 30 mg/kg, about 7 mg/kg to about 30 mg/kg, about 8 mg/kg to about 30 mg/kg, about 9 mg/kg to about 30 mg/kg, about 10 mg/kg to about 30 mg/kg, about 11 mg/kg to about 30 mg/kg, about 12 mg/kg to about 30 mg/kg, about 13 mg/kg to about 30 mg/kg, about 14 mg/kg to about 30 mg/kg, about 15 mg/kg to about 30 mg/kg, about 16 mg/kg to about 30 mg/kg, about 17 mg/kg to about 30 mg/kg, about 18 mg/kg to about 30 mg/kg, about 19 mg/kg to about 30 mg/kg, about 20 mg/kg to about 30 mg/kg, about 21 mg/kg to about 30 mg/kg, about 22 mg/kg to about 30 mg/kg, about 23 mg/kg to about 30 mg/kg, about 24 mg/kg to about 30 mg/kg, about 25 mg/kg to about 30 mg/kg, about 26 mg/kg to about 30 mg/kg, about 27 mg/kg to about 30 mg/kg, about 28 mg/kg to about 30 mg/kg, about 29 mg/kg to about 30 mg/kg, about 0.1 mg/kg to about 25 mg/kg, about 0.3 mg/kg to about 25 mg/kg, 0.9 mg/kg to about 25 mg/kg, about 1 mg/kg to about 25 mg/kg, about 2.5 mg/kg to about 25 mg/kg, about 3 mg/kg to about 25 mg/kg, about 4 mg/kg to about 25 mg/kg, about 5 mg/kg to about 25 mg/kg, about 6 mg/kg to about 25 mg/kg, about 7 mg/kg to about 25 mg/kg, about 8 mg/kg to about 25 mg/kg, about 9 mg/kg to about 25 mg/kg, about 10 mg/kg to about 25 mg/kg, about 11 mg/kg to about 25 mg/kg, about 12 mg/kg to about 25 mg/kg, about 13 mg/kg to about 25 mg/kg, about 14 mg/kg to about 25 mg/kg, about 15 mg/kg to about 25 mg/kg, about 16 mg/kg to about 25 mg/kg, about 17 mg/kg to about 25 mg/kg, about 18 mg/kg to about 25 mg/kg, about 19 mg/kg to about 25 mg/kg, about 20 mg/kg to about 25 mg/kg, about 21 mg/kg to about 25 mg/kg, about 22 mg/kg to about 25 mg/kg, about 23 mg/kg to about 25 mg/kg, about 24 mg/kg to about 25 mg/kg, about 0.1 mg/kg to about 20 mg/kg, about 0.3 mg/kg to about 20 mg/kg, 0.9 mg/kg to about 20 mg/kg, about 1 mg/kg to about 20 mg/kg, about 2.5 mg/kg to about 20 mg/kg, about 3 mg/kg to about 20 mg/kg, about 4 mg/kg to about 20 mg/kg, about 5 mg/kg to about 20 mg/kg, about 6 mg/kg to about 20 mg/kg, about 7 mg/kg to about 20 mg/kg, about 8 mg/kg to about 20 mg/kg, about 9 mg/kg to about 20 mg/kg, about 10 mg/kg to about 20 mg/kg, about 11 mg/kg to about 20 mg/kg, about 12 mg/kg to about 20 mg/kg, about 13 mg/kg to about 20 mg/kg, about 14 mg/kg to about 20 mg/kg, about 15 mg/kg to about 20 mg/kg, about 16 mg/kg to about 20 mg/kg, about 17 mg/kg to about 20 mg/kg, about 18 mg/kg to about 20 mg/kg, about 19 mg/kg to about 20 mg/kg, about 0.1 mg/kg to about 15 mg/kg, about 0.3 mg/kg to about 15 mg/kg, 0.9 mg/kg to about 15 mg/kg, about 1 mg/kg to about 15 mg/kg, about 2.5 mg/kg to about 15 mg/kg, about 3 mg/kg to about 15 mg/kg, about 4 mg/kg to about 15 mg/kg, about 5 mg/kg to about 15 mg/kg, about 6 mg/kg to about 15 mg/kg, about 7 mg/kg to about 15 mg/kg, about 8 mg/kg to about 15 mg/kg, about 9 mg/kg to about 15 mg/kg, about 10 mg/kg to about 15 mg/kg, about 11 mg/kg to about 15 mg/kg, about 12 mg/kg to about 15 mg/kg, about 13 mg/kg to about 15 mg/kg, or about 14 mg/kg to about 15 mg/kg.

In a further embodiment, the second antibody is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, or about 30 mg/kg.

In a further embodiment, the second antibody is administered at a body weight-based dose of about 25 mg/kg or 15 mg/kg.

In a further embodiment, the second antibody is administered about once every week, about once every two weeks, about once every three weeks, about once every four weeks, about once every month, about once every 3-6 months, or longer.

In a further embodiment, the second antibody is administered intravenously.

In a further embodiment, oxaliplatin is administered at a body surface area-based dose of about 10 to about 150 mg/m², about 20 to about 150 mg/m², about 30 to about 150 mg/m², about 40 to about 150 mg/m², about 50 to about 150 mg/m², about 60 to about 150 mg/m², about 70 to about 150 mg/m², about 75 to about 150 mg/m², about 80 to about 150 mg/m², about 85 to about 150 mg/m², about 90 to about 150 mg/m², about 95 to about 150 mg/m², about 100 to about 150 mg/m², about 105 to about 150 mg/m², about 110 to about 150 mg/m², about 115 to about 150 mg/m², about 120 to about 150 mg/m², about 125 to about 150 mg/m², about 130 to about 150 mg/m², about 135 to about 150 mg/m², about 140 to about 150 mg/m², about 145 to about 150 mg/m², about 10 to about 140 mg/m², about 20 to about 140 mg/m², about 30 to about 140 mg/m², about 40 to about 140 mg/m², about 50 to about 140 mg/m², about 60 to about 140 mg/m², about 70 to about 140 mg/m², about 75 to about 140 mg/m², about 80 to about 140 mg/m², about 85 to about 140 mg/m², about 90 to about 140 mg/m², about 95 to about 140 mg/m², about 100 to about 140 mg/m², about 105 to about 140 mg/m², about 110 to about 140 mg/m², about 115 to about 140 mg/m², about 120 to about 140 mg/m², about 125 to about 140 mg/m², about 130 to about 140 mg/m², about 135 to about 140 mg/m², about 10 to about 130 mg/m², about 20 to about 130 mg/m², about 30 to about 130 mg/m², about 40 to about 130 mg/m², about 50 to about 130 mg/m², about 60 to about 130 mg/m², about 70 to about 130 mg/m², about 75 to about 130 mg/m², about 80 to about 130 mg/m², about 85 to about 130 mg/m², about 90 to about 130 mg/m², about 95 to about 130 mg/m², about 100 to about 130 mg/m², about 105 to about 130 mg/m², about 110 to about 130 mg/m², about 115 to about 130 mg/m², about 120 to about 130 mg/m², or about 125 to about 130 mg/m².

In a further embodiment, oxaliplatin is administered at a body surface area-based dose of about 10 mg/m², about 20 mg/m², about 30 mg/m², about 40 mg/m², about 50 mg/m², about 60 mg/m², about 70 mg/m², about 75 mg/m², about 80 mg/m², about 85 mg/m², about 90 mg/m², about 95 mg/m², about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², or about 150 mg/m².

In a further embodiment, oxaliplatin is administered at a body surface area-based dose of about 130 mg/m².

In a further embodiment, oxaliplatin is administered about once every week, about once every two weeks, about once every three weeks, about once every four weeks, about once every month, about once every 3-6 months, or longer.

In a further embodiment, oxaliplatin is administered intravenously.

In a further embodiment, capecitabine is administered at a body surface area-based dose of about 100 to about 1200 mg/m², about 200 to about 1200 mg/m², about 300 to about 1200 mg/m², about 400 to about 1200 mg/m², about 500 to about 1200 mg/m², about 600 to about 1200 mg/m², about 650 to about 1200 mg/m², about 700 to about 1200 mg/m², about 750 to about 1200 mg/m², about 800 to about 1200 mg/m², about 850 to about 1200 mg/m², about 900 to about 1200 mg/m², about 950 to about 1200 mg/m², about 1000 to about 1200 mg/m², about 1050 to about 1200 mg/m², about 1100 to about 1200 mg/m², about 1150 to about 1200 mg/m², about 100 to about 1100 mg/m², about 200 to about 1100 mg/m², about 300 to about 1100 mg/m², about 400 to about 1100 mg/m², about 500 to about 1100 mg/m², about 600 to about 1100 mg/m², about 650 to about 1100 mg/m², about 700 to about 1100 mg/m², about 750 to about 1100 mg/m², about 800 to about 1100 mg/m², about 850 to about 1100 mg/m², about 900 to about 1100 mg/m², about 950 to about 1100 mg/m², about 1000 to about 1100 mg/m², about 100 to about 1000 mg/m², about 200 to about 1000 mg/m², about 300 to about 1000 mg/m², about 400 to about 1000 mg/m², about 500 to about 1000 mg/m², about 600 to about 1000 mg/m², about 650 to about 1000 mg/m², about 700 to about 1000 mg/m², about 750 to about 1000 mg/m², about 800 to about 1000 mg/m², about 850 to about 1000 mg/m², about 900 to about 1000 mg/m², or about 950 to about 1000 mg/m².

In a further embodiment, capecitabine is administered at a body surface area-based dose of about 100 mg/m², about 200 mg/m², about 300 mg/m², about 400 mg/m², about 500 mg/m², about 600 mg/m², about 650 mg/m², about 700 mg/m², about 750 mg/m², about 800 mg/m², about 850 mg/m², about 900 mg/m², about 950 mg/m², about 1000 mg/m², about 1050 mg/m², about 1100 mg/m², about 1150 mg/m², or about 1200 mg/m².

In a further embodiment, capecitabine is administered at a body surface area-based dose of about 1000 mg/m².

In a further embodiment, capecitabine is administered once daily, twice daily, or thrice daily.

In a further embodiment, capecitabine is administered orally.

In a further embodiment, on a body weight basis, trastuzumab is administered at an initial loading dose of about 8 mg/kg followed by a dose of about 6 mg/kg; the second antibody is administered at a body weight-based dose of about 25 mg/kg or 15 mg/kg; oxaliplatin is administered at a body surface area-based dose of about 130 mg/m²; capecitabine is administered at a body surface area-based dose of about 1000 mg/m².

In a further embodiment, the second antibody is administered to the patient before the administration of trastuzumab.

In a further embodiment, the second antibody is administered to the patient after the administration of trastuzumab.

In a further embodiment, trastuzumab and the second antibody are administered concurrently.

In a further embodiment, the method further comprises administering an immune checkpoint inhibitor.

In a further embodiment, the immune checkpoint inhibitor is an anti-PD-1 antibody or an anti-PD-L1 antibody.

In a further embodiment, the immune checkpoint inhibitor is an anti-PD-1 antibody.

In yet another aspect, the present disclosure further provides use of the second antibody in preparing a medicament for treating HER2-positive gastric cancer or gastroesophageal junction adenocarcinoma in a patient, and the treatment comprises administering to the patient a combination therapy, wherein the combination therapy comprises:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

In an alternative aspect, the present disclosure further provides use of a second antibody, trastuzumab, and a chemotherapeutic agent in preparing a medicament for treating HER2-positive gastric cancer or gastroesophageal junction adenocarcinoma in a patient, and the treatment comprises administering to the patient a combination therapy, wherein the combination therapy comprises:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

In a further embodiment, the chemotherapeutic agent is a fluorouracil drug. In a further embodiment, the chemotherapeutic agent is a platinum-based drug.

In a further embodiment, the chemotherapeutic agent is a combination of a fluorouracil drug and a platinum-based drug.

In a further embodiment, the fluorouracil drug is selected from 5-fluorouracil and capecitabine.

In a further embodiment, the platinum-based drug is selected from cisplatin and oxaliplatin. In a further embodiment, the fluorouracil drug is capecitabine. In a further embodiment, the platinum-based drug is oxaliplatin. In a further embodiment, the chemotherapeutic agent is a combination of capecitabine and oxaliplatin.

In a further embodiment, the cancer is HER2-positive advanced gastric cancer.

In a further embodiment, the cancer is unresectable or metastatic HER2-positive gastric cancer or gastroesophageal junction adenocarcinoma.

In a further embodiment, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In a further embodiment, the heavy chain of the second antibody comprises or consists of the amino acid sequence set forth in SEQ ID NO: 5, and the light chain of the second antibody comprises or consists of the amino acid sequence set forth in SEQ ID NO: 10.

In a further embodiment, trastuzumab is administered at a body weight-based dose of about 0.1 mg/kg to about 10 mg/kg.

In a further embodiment, trastuzumab is administered at a body weight-based dose of about 0.1 mg/kg to about 10 mg/kg, about 0.3 mg/kg to about 10 mg/kg, 0.9 mg/kg to about 10 mg/kg, about 1 mg/kg to about 10 mg/kg, about 2.5 mg/kg to about 10 mg/kg, about 3 mg/kg to about 10 mg/kg, about 4 mg/kg to about 10 mg/kg, about 5 mg/kg to about 10 mg/kg, about 6 mg/kg to about 10 mg/kg, about 7 mg/kg to about 10 mg/kg, about 8 mg/kg to about 10 mg/kg, about 9 mg/kg to about 10 mg/kg, about 0.1 mg/kg to about 8 mg/kg, about 0.3 mg/kg to about 8 mg/kg, 0.9 mg/kg to about 8 mg/kg, about 1 mg/kg to about 8 mg/kg, about 2.5 mg/kg to about 8 mg/kg, about 3 mg/kg to about 8 mg/kg, about 4 mg/kg to about 8 mg/kg, about 5 mg/kg to about 8 mg/kg, about 6 mg/kg to about 8 mg/kg, about 7 mg/kg to about 8 mg/kg, about 0.1 mg/kg to about 6 mg/kg, about 0.3 mg/kg to about 6 mg/kg, 0.9 mg/kg to about 6 mg/kg, about 1 mg/kg to about 6 mg/kg, about 2.5 mg/kg to about 6 mg/kg, about 3 mg/kg to about 6 mg/kg, about 4 mg/kg to about 6 mg/kg, or about 5 mg/kg to about 6 mg/kg.

In a further embodiment, trastuzumab is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

In a further embodiment, on a body weight basis, trastuzumab is administered at an initial loading dose of about 8 mg/kg followed by a dose of about 6 mg/kg.

In a further embodiment, trastuzumab is administered about once every week, about once every two weeks, about once every three weeks, about once every four weeks, about once every month, about once every 3-6 months, or longer.

In a further embodiment, trastuzumab is administered intravenously.

In a further embodiment, the second antibody is administered at a body weight-based dose of about 0.1 mg/kg to about 30 mg/kg, about 0.3 mg/kg to about 30 mg/kg, 0.9 mg/kg to about 30 mg/kg, about 1 mg/kg to about 30 mg/kg, about 2.5 mg/kg to about 30 mg/kg, about 3 mg/kg to about 30 mg/kg, about 4 mg/kg to about 30 mg/kg, about 5 mg/kg to about 30 mg/kg, about 6 mg/kg to about 30 mg/kg, about 7 mg/kg to about 30 mg/kg, about 8 mg/kg to about 30 mg/kg, about 9 mg/kg to about 30 mg/kg, about 10 mg/kg to about 30 mg/kg, about 11 mg/kg to about 30 mg/kg, about 12 mg/kg to about 30 mg/kg, about 13 mg/kg to about 30 mg/kg, about 14 mg/kg to about 30 mg/kg, about 15 mg/kg to about 30 mg/kg, about 16 mg/kg to about 30 mg/kg, about 17 mg/kg to about 30 mg/kg, about 18 mg/kg to about 30 mg/kg, about 19 mg/kg to about 30 mg/kg, about 20 mg/kg to about 30 mg/kg, about 21 mg/kg to about 30 mg/kg, about 22 mg/kg to about 30 mg/kg, about 23 mg/kg to about 30 mg/kg, about 24 mg/kg to about 30 mg/kg, about 25 mg/kg to about 30 mg/kg, about 26 mg/kg to about 30 mg/kg, about 27 mg/kg to about 30 mg/kg, about 28 mg/kg to about 30 mg/kg, about 29 mg/kg to about 30 mg/kg, about 0.1 mg/kg to about 25 mg/kg, about 0.3 mg/kg to about 25 mg/kg, 0.9 mg/kg to about 25 mg/kg, about 1 mg/kg to about 25 mg/kg, about 2.5 mg/kg to about 25 mg/kg, about 3 mg/kg to about 25 mg/kg, about 4 mg/kg to about 25 mg/kg, about 5 mg/kg to about 25 mg/kg, about 6 mg/kg to about 25 mg/kg, about 7 mg/kg to about 25 mg/kg, about 8 mg/kg to about 25 mg/kg, about 9 mg/kg to about 25 mg/kg, about 10 mg/kg to about 25 mg/kg, about 11 mg/kg to about 25 mg/kg, about 12 mg/kg to about 25 mg/kg, about 13 mg/kg to about 25 mg/kg, about 14 mg/kg to about 25 mg/kg, about 15 mg/kg to about 25 mg/kg, about 16 mg/kg to about 25 mg/kg, about 17 mg/kg to about 25 mg/kg, about 18 mg/kg to about 25 mg/kg, about 19 mg/kg to about 25 mg/kg, about 20 mg/kg to about 25 mg/kg, about 21 mg/kg to about 25 mg/kg, about 22 mg/kg to about 25 mg/kg, about 23 mg/kg to about 25 mg/kg, about 24 mg/kg to about 25 mg/kg, about 0.1 mg/kg to about 20 mg/kg, about 0.3 mg/kg to about 20 mg/kg, 0.9 mg/kg to about 20 mg/kg, about 1 mg/kg to about 20 mg/kg, about 2.5 mg/kg to about 20 mg/kg, about 3 mg/kg to about 20 mg/kg, about 4 mg/kg to about 20 mg/kg, about 5 mg/kg to about 20 mg/kg, about 6 mg/kg to about 20 mg/kg, about 7 mg/kg to about 20 mg/kg, about 8 mg/kg to about 20 mg/kg, about 9 mg/kg to about 20 mg/kg, about 10 mg/kg to about 20 mg/kg, about 11 mg/kg to about 20 mg/kg, about 12 mg/kg to about 20 mg/kg, about 13 mg/kg to about 20 mg/kg, about 14 mg/kg to about 20 mg/kg, about 15 mg/kg to about 20 mg/kg, about 16 mg/kg to about 20 mg/kg, about 17 mg/kg to about 20 mg/kg, about 18 mg/kg to about 20 mg/kg, about 19 mg/kg to about 20 mg/kg, about 0.1 mg/kg to about 15 mg/kg, about 0.3 mg/kg to about 15 mg/kg, 0.9 mg/kg to about 15 mg/kg, about 1 mg/kg to about 15 mg/kg, about 2.5 mg/kg to about 15 mg/kg, about 3 mg/kg to about 15 mg/kg, about 4 mg/kg to about 15 mg/kg, about 5 mg/kg to about 15 mg/kg, about 6 mg/kg to about 15 mg/kg, about 7 mg/kg to about 15 mg/kg, about 8 mg/kg to about 15 mg/kg, about 9 mg/kg to about 15 mg/kg, about 10 mg/kg to about 15 mg/kg, about 11 mg/kg to about 15 mg/kg, about 12 mg/kg to about 15 mg/kg, about 13 mg/kg to about 15 mg/kg, or about 14 mg/kg to about 15 mg/kg.

In a further embodiment, the second antibody is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, or about 30 mg/kg.

In a further embodiment, the second antibody is administered at a body weight-based dose of about 25 mg/kg or 15 mg/kg.

In a further embodiment, the second antibody is administered about once every week, about once every two weeks, about once every three weeks, about once every four weeks, about once every month, about once every 3-6 months, or longer.

In a further embodiment, the second antibody is administered intravenously.

In a further embodiment, oxaliplatin is administered at a body surface area-based dose of about 10 to about 150 mg/m², about 20 to about 150 mg/m², about 30 to about 150 mg/m², about 40 to about 150 mg/m², about 50 to about 150 mg/m², about 60 to about 150 mg/m², about 70 to about 150 mg/m², about 75 to about 150 mg/m², about 80 to about 150 mg/m², about 85 to about 150 mg/m², about 90 to about 150 mg/m², about 95 to about 150 mg/m², about 100 to about 150 mg/m², about 105 to about 150 mg/m², about 110 to about 150 mg/m², about 115 to about 150 mg/m², about 120 to about 150 mg/m², about 125 to about 150 mg/m², about 130 to about 150 mg/m², about 135 to about 150 mg/m², about 140 to about 150 mg/m², about 145 to about 150 mg/m², about 10 to about 140 mg/m², about 20 to about 140 mg/m², about 30 to about 140 mg/m², about 40 to about 140 mg/m², about 50 to about 140 mg/m², about 60 to about 140 mg/m², about 70 to about 140 mg/m², about 75 to about 140 mg/m², about 80 to about 140 mg/m², about 85 to about 140 mg/m², about 90 to about 140 mg/m², about 95 to about 140 mg/m², about 100 to about 140 mg/m², about 105 to about 140 mg/m², about 110 to about 140 mg/m², about 115 to about 140 mg/m², about 120 to about 140 mg/m², about 125 to about 140 mg/m², about 130 to about 140 mg/m², about 135 to about 140 mg/m², about 10 to about 130 mg/m², about 20 to about 130 mg/m², about 30 to about 130 mg/m², about 40 to about 130 mg/m², about 50 to about 130 mg/m², about 60 to about 130 mg/m², about 70 to about 130 mg/m², about 75 to about 130 mg/m², about 80 to about 130 mg/m², about 85 to about 130 mg/m², about 90 to about 130 mg/m², about 95 to about 130 mg/m², about 100 to about 130 mg/m², about 105 to about 130 mg/m², about 110 to about 130 mg/m², about 115 to about 130 mg/m², about 120 to about 130 mg/m², or about 125 to about 130 mg/m².

In a further embodiment, oxaliplatin is administered at a body surface area-based dose of about 10 mg/m², about 20 mg/m², about 30 mg/m², about 40 mg/m², about 50 mg/m², about 60 mg/m², about 70 mg/m², about 75 mg/m², about 80 mg/m², about 85 mg/m², about 90 mg/m², about 95 mg/m², about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², or about 150 mg/m².

In a further embodiment, oxaliplatin is administered at a body surface area-based dose of about 130 mg/m².

In a further embodiment, oxaliplatin is administered about once every week, about once every two weeks, about once every three weeks, about once every four weeks, about once every month, about once every 3-6 months, or longer.

In a further embodiment, oxaliplatin is administered intravenously.

In a further embodiment, capecitabine is administered at a body surface area-based dose of about 100 to about 1200 mg/m², about 200 to about 1200 mg/m², about 300 to about 1200 mg/m², about 400 to about 1200 mg/m², about 500 to about 1200 mg/m², about 600 to about 1200 mg/m², about 650 to about 1200 mg/m², about 700 to about 1200 mg/m², about 750 to about 1200 mg/m², about 800 to about 1200 mg/m², about 850 to about 1200 mg/m², about 900 to about 1200 mg/m², about 950 to about 1200 mg/m², about 1000 to about 1200 mg/m², about 1050 to about 1200 mg/m², about 1100 to about 1200 mg/m², about 1150 to about 1200 mg/m², about 100 to about 1100 mg/m², about 200 to about 1100 mg/m², about 300 to about 1100 mg/m², about 400 to about 1100 mg/m², about 500 to about 1100 mg/m², about 600 to about 1100 mg/m², about 650 to about 1100 mg/m², about 700 to about 1100 mg/m², about 750 to about 1100 mg/m², about 800 to about 1100 mg/m², about 850 to about 1100 mg/m², about 900 to about 1100 mg/m², about 950 to about 1100 mg/m², about 1000 to about 1100 mg/m², about 100 to about 1000 mg/m², about 200 to about 1000 mg/m², about 300 to about 1000 mg/m², about 400 to about 1000 mg/m², about 500 to about 1000 mg/m², about 600 to about 1000 mg/m², about 650 to about 1000 mg/m², about 700 to about 1000 mg/m², about 750 to about 1000 mg/m², about 800 to about 1000 mg/m², about 850 to about 1000 mg/m², about 900 to about 1000 mg/m², or about 950 to about 1000 mg/m².

In a further embodiment, capecitabine is administered at a body surface area-based dose of about 100 mg/m², about 200 mg/m², about 300 mg/m², about 400 mg/m², about 500 mg/m², about 600 mg/m², about 650 mg/m², about 700 mg/m², about 750 mg/m², about 800 mg/m², about 850 mg/m², about 900 mg/m², about 950 mg/m², about 1000 mg/m², about 1050 mg/m², about 1100 mg/m², about 1150 mg/m², or about 1200 mg/m².

In a further embodiment, capecitabine is administered at a body surface area-based dose of about 1000 mg/m².

In a further embodiment, capecitabine is administered once daily, twice daily, or thrice daily.

In a further embodiment, capecitabine is administered orally.

In a further embodiment, on a body weight basis, trastuzumab is administered at an initial loading dose of about 8 mg/kg followed by a dose of about 6 mg/kg; the second antibody is administered at a body weight-based dose of about 25 mg/kg or 15 mg/kg; oxaliplatin is administered at a body surface area-based dose of about 130 mg/m²; capecitabine is administered at a body surface area-based dose of about 1000 mg/m².

In a further embodiment, the second antibody is administered to the patient before the administration of trastuzumab.

In a further embodiment, the second antibody is administered to the patient after the administration of trastuzumab.

In a further embodiment, trastuzumab and the second antibody are administered concurrently.

In a further embodiment, the treatment further comprises administering an immune checkpoint inhibitor.

In a further embodiment, the immune checkpoint inhibitor is an anti-PD-1 antibody or an anti-PD-L1 antibody.

In a further embodiment, the immune checkpoint inhibitor is an anti-PD-1 antibody.

In some embodiments of the present disclosure, the patient has not been subjected to treatment, including, for example, having not been subjected to any systemic anti-tumor therapy for advanced unresectable or metastatic HER2-positive gastric cancer or gastroesophageal junction adenocarcinoma, or having previously received adjuvant therapy, with an interval of ≥ 6 months between the end of the last treatment and the first administration of the treatment regimen of the present disclosure, or having received treatment with an anti-tumor traditional Chinese medicine or Chinese patent medicine, with the end of the last treatment occurring before the randomization of the study involved in the present disclosure.

In some embodiments of the present disclosure, the trastuzumab, and the second anti-HER2 antibody or the binding fragment thereof is administered intravenously every 3 weeks.

In some embodiments, the second anti-HER2 antibody is administered at a dose of about 25 mg/kg. In some embodiments, the second anti-HER2 antibody is administered at a dose of about 15mg/kg. In some embodiments, trastuzumab is administered at an initial loading dose of about 8 mg/kg followed by doses of about 6 mg/kg once every 3 weeks.

In some embodiments of the present disclosure, the chemotherapeutic agent is a combination of capecitabine and oxaliplatin.

In some embodiments, oxaliplatin is administered at a dose of about 130 mg/m² via intravenous infusion once every three weeks, typically for 2-6 h on the first day of each cycle. In certain cases, for example, when the body surface area exceeds 2.0 m², the dose can be calculated by the investigator according to clinical practice.

In some embodiments, capecitabine is orally administered at a dose of about 1000 mg/m² twice daily in cycles of three weeks, typically from the first day to the fourteenth day of each cycle. In certain cases, the dose of capecitabine is calculated by the investigator according to clinical practice. For example, when the calculated theoretical dose exceeds 1500 mg, the dose of capecitabine is selected to be about 1500 mg.

Immune checkpoint inhibitors are also used as an important part of the treatment regimen for the treatment of advanced gastric cancer. For example, in the phase III KEYNOTE-811 study on the treatment of HER2-positive gastric cancer, compared to the combination of trastuzumab and a chemotherapeutic agent, further combination with pembrolizumab demonstrated a significantly improved response rate. Moreover, the FDA has approved the treatment regimen of pembrolizumab in combination with trastuzumab, fluorouracil and a platinum-based chemotherapeutic agent for the first-line treatment of a patient with advanced unresectable or metastatic HER2-positive gastric cancer or gastroesophageal junction adenocarcinoma. The combined treatment regimen of trastuzumab + a second anti-HER2 antibody + a chemotherapeutic agent involved in the present disclosure may also be used in combination with an immune checkpoint inhibitor to increase the response rate. In some embodiments of the present disclosure, in the use or treatment regimen involved in the present disclosure, an immune checkpoint inhibitor, preferably an anti-PD-1 antibody or an anti-PD-L1 antibody, preferably an anti-PD-1 antibody, such as nivolumab, pembrolizumab, sintilimab, tislelizumab, camrelizumab, toripalimab, or slulimab, can be administered concurrently with or at intervals from the combined treatment regimen.

### V. Pharmaceutical combination/pharmaceutical composition

The present disclosure provides a pharmaceutical combination, which relates to the combination of trastuzumab and the second anti-HER2 antibody described above, and specifically comprises trastuzumab and a second anti-HER2 antibody or a binding fragment thereof.

The present disclosure further provides a pharmaceutical composition, e.g., a pharmaceutical composition for treating gastric cancer, comprising trastuzumab, the second anti-HER2 antibody described above, and a pharmaceutically acceptable carrier.

In some embodiments, the pharmaceutical composition described herein is in the form of a liquid formulation or a lyophilized powder, typically in the form of a liquid formulation. The formulation comprises antibodies (trastuzumab and the second anti-HER2 antibody), a surfactant (e.g., polysorbate 20 or 80), a buffer (histidine buffer, acetate buffer, citrate buffer, or a mixture of two buffers), and a stabilizer (e.g., sugar, alcohol, salt, amino acid, etc.), at a suitable pH value.

In one aspect, the present disclosure further provides a combination product, comprising: trastuzumab;
a second antibody, wherein the second antibody is a second anti-HER2 antibody or an antigen-binding fragment thereof having a different binding site from trastuzumab, comprising:
a heavy chain variable region, comprising the HCDR1 set forth in SEQ ID NO: 1, the HCDR2 set forth in SEQ ID NO: 2, and the HCDR3 set forth in SEQ ID NO: 3, and
a light chain variable region, comprising the LCDR1 set forth in SEQ ID NO: 6, the LCDR2 set forth in SEQ ID NO: 7, and the LCDR3 set forth in SEQ ID NO: 8; and
a chemotherapeutic agent.

In a further embodiment, the chemotherapeutic agent is a fluorouracil drug. In a further embodiment, the chemotherapeutic agent is a platinum-based drug.

In a further embodiment, the chemotherapeutic agent is a combination of a fluorouracil drug and a platinum-based drug.

In a further embodiment, the fluorouracil drug is selected from 5-fluorouracil and capecitabine. In a further embodiment, the platinum-based drug is selected from cisplatin and oxaliplatin. In a further embodiment, the fluorouracil drug is capecitabine. In a further embodiment, the platinum-based drug is oxaliplatin. In a further embodiment, the chemotherapeutic agent is a combination of capecitabine and oxaliplatin.

In a further embodiment, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In a further embodiment, the heavy chain of the second antibody comprises or consists of the amino acid sequence set forth in SEQ ID NO: 5, and the light chain of the second antibody comprises or consists of the amino acid sequence set forth in SEQ ID NO: 10.

In a further embodiment, the combination product further comprises an anti-PD-1 antibody and/or an anti-PD-L1 antibody.

In a further embodiment, the combination product further comprises an anti-PD-1 antibody.

### VI. Kit

The present disclosure further relates to a kit for the above treatment of gastric cancer, which typically comprises antibodies, a chemotherapeutic agent, and/or a label and package insert for intended use. Specifically, the present disclosure relates to a kit for the treatment of gastric cancer, especially for the treatment of advanced unresectable or metastatic HER2-positive gastric cancer, comprising the doses of trastuzumab, a second anti-HER2 antibody, and a chemotherapeutic agent for at least one cycle. In some exemplary embodiments, trastuzumab, the second anti-HER2 antibody, and/or the chemotherapeutic agent are packaged in a unit dosage form. The second anti-HER2 antibody is as defined above. In a preferred embodiment of the present disclosure, the chemotherapeutic agent in the kit is a fluorouracil compound and a platinum-based drug, for example, the fluorouracil compound is 5-fluorouracil or capecitabine, and the platinum-based drug is cisplatin or oxaliplatin. In a more preferred embodiment, the kit comprises trastuzumab, the second anti-HER2 antibody, capecitabine, and oxaliplatin.

Specifically, in one aspect, the present disclosure further provides a kit for treating HER2-positive gastric cancer or gastroesophageal junction adenocarcinoma, comprising:
trastuzumab;
a second antibody, wherein the second antibody is a second anti-HER2 antibody or an antigen-binding fragment thereof having a different binding site from trastuzumab, comprising:
   a heavy chain variable region, comprising the HCDR1 set forth in SEQ ID NO: 1, the HCDR2 set forth in SEQ ID NO: 2, and the HCDR3 set forth in SEQ ID NO: 3, and
   a light chain variable region, comprising the LCDR1 set forth in SEQ ID NO: 6, the LCDR2 set forth in SEQ ID NO: 7, and the LCDR3 set forth in SEQ ID NO: 8; and
   a chemotherapeutic agent.

In a further embodiment, the chemotherapeutic agent is a fluorouracil drug.

In a further embodiment, the chemotherapeutic agent is a platinum-based drug.

In a further embodiment, the chemotherapeutic agent is a combination of a fluorouracil drug and a platinum-based drug.

In a further embodiment, the fluorouracil drug is selected from 5-fluorouracil and capecitabine. In a further embodiment, the platinum-based drug is selected from cisplatin and oxaliplatin.

In a further embodiment, the fluorouracil drug is capecitabine.

In a further embodiment, the platinum-based drug is oxaliplatin.

In a further embodiment, the chemotherapeutic agent is a combination of capecitabine and oxaliplatin.

In a further embodiment, the cancer is HER2-positive advanced gastric cancer.

In a further embodiment, the cancer is unresectable or metastatic HER2-positive gastric cancer or gastroesophageal junction adenocarcinoma.

In a further embodiment, the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

In a further embodiment, the heavy chain of the second antibody comprises or consists of the amino acid sequence set forth in SEQ ID NO: 5, and the light chain of the second antibody comprises or consists of the amino acid sequence set forth in SEQ ID NO: 10.

In a further embodiment, the kit further comprises a package insert.

In a further embodiment, the kit further comprises an anti-PD-1 antibody and/or an anti-PD-L1 antibody.

In a further embodiment, the kit further comprises an anti-PD-1 antibody.

The sequence information involved in the present disclosure is summarized in Table 1.

**Table 1. Sequence listing**

| SEQ ID NO: | Name | Amino acid sequence |
|---|---|---|
| 1 | Second anti-HER2 antibody_HCDR1 | GFTFSSYTMS |
| 2 | Second anti-HER2 antibody_HCDR2 | WVAYISAGGGSTYYPDTVKG |
| 3 | Second anti-HER2 antibody_HCDR3 | ARHLGGTASFDY |
| 4 | Second anti-HER2 antibody_heavy chain variable region | EVQLVESGGGLVQPGGSLRLSCAASGFTFSSYTMSWVRQAP GKGLEWVAYISAGGGSTYYPDTVKGRFTISRDNAKNSLYLQ MNSLRAEDTAVYYCARHLGGTASFDYWGQGTLVTVSS |
| 5 | Second anti-HER2 antibody_heavy chain | EVQLVESGGGLVQPGGSLRLSCAASGFTFSSYTMSWVRQAP GKGLEWVAYISAGGGSTYYPDTVKGRFTISRDNAKNSLYLQ MNSLRAEDTAVYYCARHLGGTASFDYWGQGTLVTVSSASTK GPSVFPLAPSSKSTSGGTAALGCLVKDYFPEPVTVSWNSGAL TSGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |
| 6 | Second anti-HER2 antibody_LCDR1 | LASQTIGTWLAWY |
| 7 | Second anti-HER2 antibody_LCDR2 | LLIYVATSLAD |
| 8 | Second anti-HER2 antibody_LCDR3 | QQNAYAPWT |
| 9 | Second anti-HER2 antibody_light chain variable region | DIQMTQSPSSLSASVGDRVTITCLASQTIGTWLAWYQQKPGK APKLLIYVATSLADGVPSRFSGSGSGTDFTLTISSLQPEDFATY YCQQNAYAPWTFGQGTKVEIKRTV |
| 10 | Second anti-HER2 antibody_light chain | DIQMTQSPSSLSASVGDRVTITCLASQTIGTWLAWYQQKPGK APKLLIYVATSLADGVPSRFSGSGSGTDFTLTISSLQPEDFATY YCQQNAYAPWTFGQGTKVEIKRTVAAPSVFIFPPSDEQLKSG TASVVCLLNNFYPREAKVQWKVDNALQSGNSQESVTEQDS KDSTYSLSSTLTLSKADYEKHKVYACEVTHQGLSSPVTKSFN RGEC |
| 11 | Trastuzumab_heavy chain | EVQLVESGGGLVQPGGSLRLSCAASGFNIKDTYIHWVRQAP GKGLEWVARIYPTNGYTRYADSVKGRFTISADTSKNTAYLQ MNSLRAEDTAVYYCSRWGGDGFYAMDYWGQGTLVTVSSAS TKGPSVFPLAPSSKSTSGGTAALGCLVKDYFPEPVTVSWNSG ALTSGVHTFPAVLQSSGLYSLSSVVTVPSSSLGTQTYICNVNH KPSNTKVDKKVEPKSCDKTHTCPPCPAPELLGGPSVFLFPPKP KDTLMISRTPEVTCVVVDVSHEDPEVKFNWYVDGVEVHNA KTKPREEQYNSTYRVVSVLTVLHQDWLNGKEYKCKVSNKA LPAPIEKTISKAKGQPREPQVYTLPPSREEMTKNQVSLTCLVK GFYPSDIAVEWESNGQPENNYKTTPPVLDSDGSFFLYSKLTV DKSRWQQGNVFSCSVMHEALHNHYTQKSLSLSPGK |
| 12 | Trastuzumab_light chain | DIQMTQSPSSLSASVGDRVTITCRASQDVNTAVAWYQQKPGK APKLLIYSASFLYSGVPSRFSGSRSGTDFTLTISSLQPEDFATY YCQQHYTTPPTFGQGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFNR GEC |
| 13 | Pertuzumab_heavy chain | EVQLVESGGGLVQPGGSLRLSCAASGFTFTDYTMDWVRQAP GKGLEWVADVNPNSGGSIYNQRFKGRFTLSVDRSKNTLYLQ MNSLRAEDTAVYYCARNLGPSFYFDYWGQGTLVTVSSASTK GPSVFPLAPSSKSTSGGTAALGCLVKDYFPEPVTVSWNSGAL TSGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSREEM TKNQVSLTCLVKGFYPSDIAVEWESNGQPENNYKTTPPVLDS DGSFFLYSKLTVDKSRWQQGNVFSCSVMHEALHNHYTQKSL SLSPG |
| 14 | Pertuzumab_light chain | DIQMTQSPSSLSASVGDRVTITCKASQDVSIGVAWYQQKPGK APKLLIYSASYRYTGVPSRFSGSGSGTDFTLTISSLQPEDFATY YCQQYYIYPYTFGQGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFNR GEC |

**Table 2. List of abbreviations**

| Abbreviation | Full version | Chinese definition |
|---|---|---|
| AE | Adverse Event | 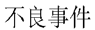 |
| CNS | Central Nervous System | 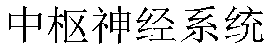 |
| CR | Complete response | 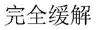 |
| CSCO | Chinese Society of Clinical Oncology | 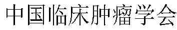 |
| CTLA-4 | Cytotoxic T-lymphocyte-associated protein 4 | 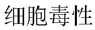 T 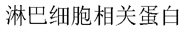 4 |
| DOR | Duration of response | 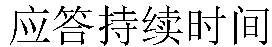 |
| ESMO | European Society for Medical Oncology | 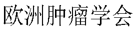 |
| FDA | Food and Drug Administration | 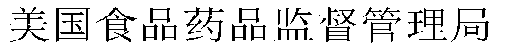 |
| FISH | Fluorescence in situ hybridization | 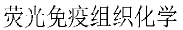 |
| HBV | Hepatitis B virus | 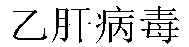 |
| HCV | Hepatitis C virus | 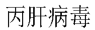 |
| HER2 | Human Epidermal Growth Factor Receptor 2 | 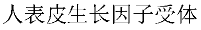 |
| HR | Hazard ratio | 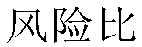 |
| ICF | Informed Consetn Form | 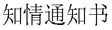 |
| IDMC | Indepenent Data Monitoring Committee | 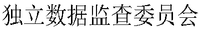 |
| IHC | Immunohistorhemistry | 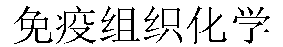 |
| IRRC | Immune-related response criteria | 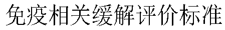 |
| ITT | Intention to Treat | 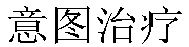 |
| IV | Intravenous | 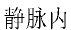 |
| NCCN | National Comprehensive Cancer Network | 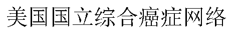 |
| NYHA | New York Heart Association | 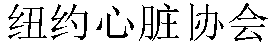 |
| ORR | Overall response rate | 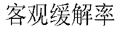 |
| OS | Overall Surviva | 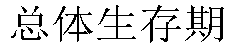 |
| PD-1 | Programmed cell death 1 | 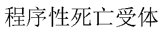 -1 |
| PD-L1 | Programmed cell death ligand-1 | 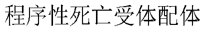 -1 |
| PDX | Patient-derivedtumor xenograft | 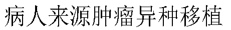 |
| PFS | Progreassion Free Survival | 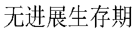 |
| PR | Partical response | 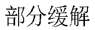 |
| RECIST | Response evaluation Criteria in Solid | 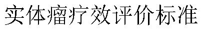 |
| TTR | Time to response | 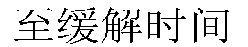 |
| TEAE | Treatment emergent adverse event | 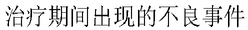 |
| XELOX | Xeloda plus Oxaliplatin | 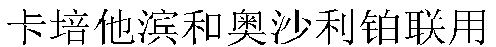 |

Source of drugs: Trastuzumab (or biosimilar): from Henlius, under the trade name Hanquyou. Second anti-HER2 antibody: prepared according to the method in Chinese Patent Application No. 201480024152.3, where the antibody designated as hz1E11-133 was the reference antibody; Pertuzumab: prepared according to the methods described in WO01/00245 and WO2006/007398, or using a commercially available product or a biosimilar approved for clinical study;
Chemotherapeutic agent:
capecitabine and oxaliplatin, Qilu Pharmaceutical;
Cells for the experiments: gastric cancer cells NCI-N87, purchased from the National Cancer Institute (NCI); SNU216, purchased from CoBioer.
HER2-Fc protein used in Example 1, Sino Biological (PA, USA), Cat. No. 10004-H02H, LC10JA1203.

The present disclosure is illustrated by the following non-limiting examples.

### Example 1. Study on HER2 epitopes of trastuzumab and second anti-HER2 antibodies

The epitope binding competition among trastuzumab, the second anti-HER2 antibody, and pertuzumab was analyzed. The second anti-HER2 antibody was immobilized on a Biacore CM5 sensor chip using amine coupling of about 1000 RU, and then (a) an HER2-Fc protein, trastuzumab, and pertuzumab, and (b) an HER2-Fc protein, pertuzumab, and trastuzumab, were sequentially added.

Referring to the HER2 epitope affinity study shown in FIG. 1, the second anti-HER2 antibody exhibited no competitive binding with trastuzumab and pertuzumab, and the second anti-HER2 antibody has a different binding site from that of trastuzumab. Although the second anti-HER2 antibody also binds to HER2 subdomain IV, it does not compete with trastuzumab.

### Example 2. Study on HER2-mediated endocytosis capacity and EGFR cell surface expression level of gastric cancer cells NCI-N87 and SNU216 treated with combination of trastuzumab and second anti-HER2 antibody

### Endocytosis capacity study

The time gradient change in the endocytosis of the HER2 antibody in the gastric cancer cells NCI-N87 and SNU216 treated with trastuzumab, the second anti-HER2 antibody, pertuzumab, and a combination thereof was observed by PHrodo Green staining. A HER2 internalization assay was performed using pHrodoiFL Green STP ester amine reactive dye (P35369, Invitrogen). NCI-N87 and SNU216 gastric cancer cells were seeded in a 96-well plate (CLS3799, Corning) at 1.5 × 10⁵ cells/well and then co-incubated with trastuzumab, anti-HER2 antibody, pertuzumab, trastuzumab + pertuzumab, or trastuzumab + anti-HER2 antibody (10 µg/mL) on ice for 1 h. After a short wash with a wash buffer to remove unbound antibodies, one group of cells was placed on ice for freezing to stop internalization, and the remaining cells were placed in a 37 °C incubator for further incubation. After the co-incubation with the antibody, the cells were fixed with a fixation buffer (420801, Biolegend) for 30 min. Finally, all stained cells were washed with a staining buffer and analyzed on the CytoFLEX LX flow cytometer (Beckman Coulter, USA).

Referring to FIG. 2, trastuzumab + pertuzumab exhibited limited HER2 internalization in gastric cancer cells NCI-N87 and SNU216, while trastuzumab + anti-HER2 antibody induced stronger HER2 internalization in the NCI-N87 and SNU216 gastric cancer cells. The anti-HER2 antibody and trastuzumab in the combination simultaneously bind to subdomain IV of HER2, while trastuzumab and pertuzumab in the combination bind to subdomain IV and subdomain II of HER2. Therefore, the antibodies binding to different subdomains will result in different internalization rates of the receptor, suggesting a unique synergistic effect of the combination of the anti-HER2 antibody and trastuzumab.

### EGFR cell expression level study

The expression levels of EGFR and HER2 proteins were detected using BV421 anti-human EGF receptor (749755, BD) and AF647 anti-human CD340 (erbB2/HER-2; 324412, Biolegend). All stained cells were analyzed on the CytoFLEX LX flow cytometer (Beckman Coulter, USA). Data were analyzed using FlowJo software.

Referring to FIG. 3, in NCI-N87 gastric cancer cells, the combination treatment with the anti-HER2 antibody and trastuzumab not only reduced the expression of HER2 (a, b, and c in FIG. 3), but also reduced the expression of EGFR on the cell surface (d, e, and f in FIG. 3), indicating that EGFR was internalized together with HER2 in the form of the HER2/EGFR heterodimer, while the combination treatment with pertuzumab and trastuzumab induced a reduction in the expression of HER2 but limited reduction in the expression of EGFR on the cell surface.

### Example 3. Study on synergistic effect of combination of trastuzumab and second anti-HER2 antibody on treating HER2-positive gastric cancer

The synergistic anti-tumor effect of trastuzumab and the second anti-HER2 antibody was investigated through the viability of gastric cancer cells NCI-N87 and a PDX model of HER2-positive gastric cancer.

After a 72-h treatment with trastuzumab, the second anti-HER2 antibody, pertuzumab, the combination of trastuzumab/second anti-HER2 antibody, or the combination of trastuzumab/pertuzumab, the viability of gastric cancer NCI-N87 cells was determined. The apoptosis was assessed by measuring the activity of caspases 3/7 in gastric cancer NCI-N87 cells using the Caspase-Glo^{®}3/7 Assay system. A xenograft human gastric cancer NCI-N87 model and a human gastric cancer PDX model were treated with the antibodies at given doses. All data are presented as mean ± SEM. The p values were calculated using Student's t test. ** denotes p < 0.01, and * denotes p < 0.05.

### In vitro proliferation study

The cell proliferation was determined using the CellTiter-Glo luminescent cell viability kit (G7571, Promega). The cells were seeded in a 96-well plate (CLS3799, Corning) at 1.5 × 10⁴ cells/well, incubated overnight at 37 °C, and separately treated with IgG1, trastuzumab, second anti-HER2 antibody, pertuzumab, trastuzumab/second anti-HER2 antibody, or trastuzumab/pertuzumab. Meanwhile, the luminescence value of the plate at T = 0 h was measured. After 72 h of incubation, the luminescence values in the plate at T = 72 h were measured using Spark (Tecan, Switzerland). The cell viability was calculated as follows: cell viability = (T72 - T0)/(T72(c) - T0) × 100%, where, T: treatment, and c: control.

### Apoptosis assay

The apoptosis was assessed by measuring the activity of caspases 3/7 in NCI-N87 cells using the Caspase-Glo^{®}3/7 Assay (G8093, Promega) system. The cells were seeded in a 96-well plate (CLS3799, Corning) at about 1.5 × 10⁴ cells/well and incubated overnight. The original medium was replaced with a drug-containing culture medium of 100 µL, and the cells were treated for 24 h. After an incubation at room temperature for 30 min, 100 µL of Caspase-Glo 3/7 reagent was added. The plate was incubated in the dark for 30 min and detected using Spark (Tecan, Switzerland) after gentle shaking.

### Animal tumor model study

In the xenograft human gastric cancer NCI-N87 model, BALB/c n nude female mice were subcutaneously grafted with 5 × 10⁶ NCI-N87 cells. When the mean tumor volume of the mice reached about 120-150 mm³, the mice were randomized and then treated as described below. The second anti-HER2 antibody or the control reagent was intraperitoneally administered at 10 mg/kg twice every week for 28 consecutive days, with 8 mice in each group. For the PDX model, tumor xenografts were extracted from the gastric cancer tissues in patients and grafted subcutaneously into the right back of the NCG mice. The PDX model was treated with trastuzumab, trastuzumab/second anti-HER2 antibody, and trastuzumab/pertuzumab at different doses twice every week for 52-60 consecutive days, with n = 5 mice in each group. Tumor volume and body weight were measured twice every week.

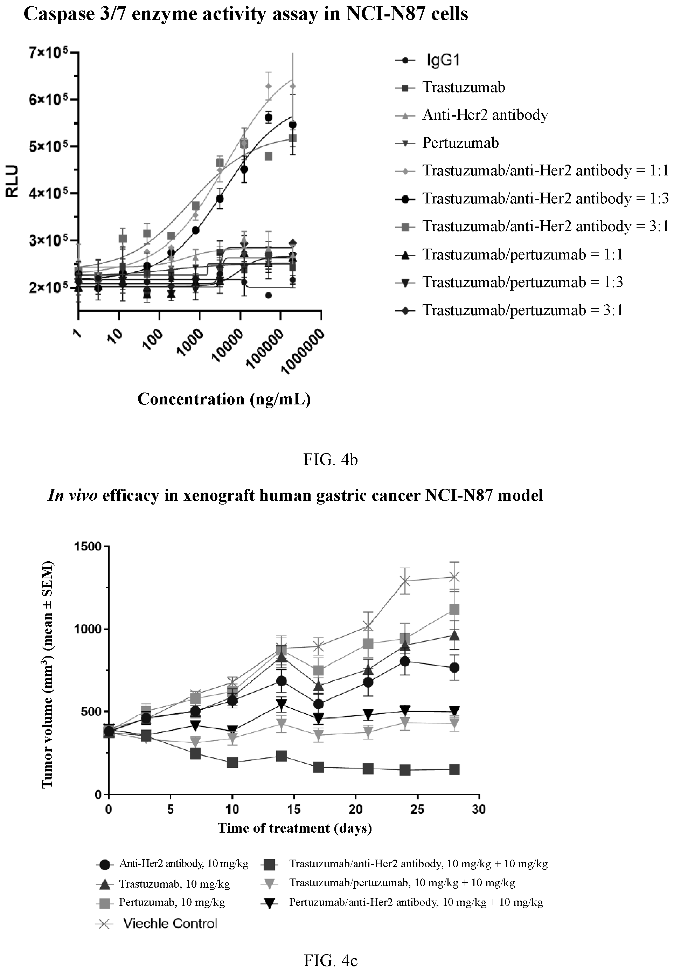

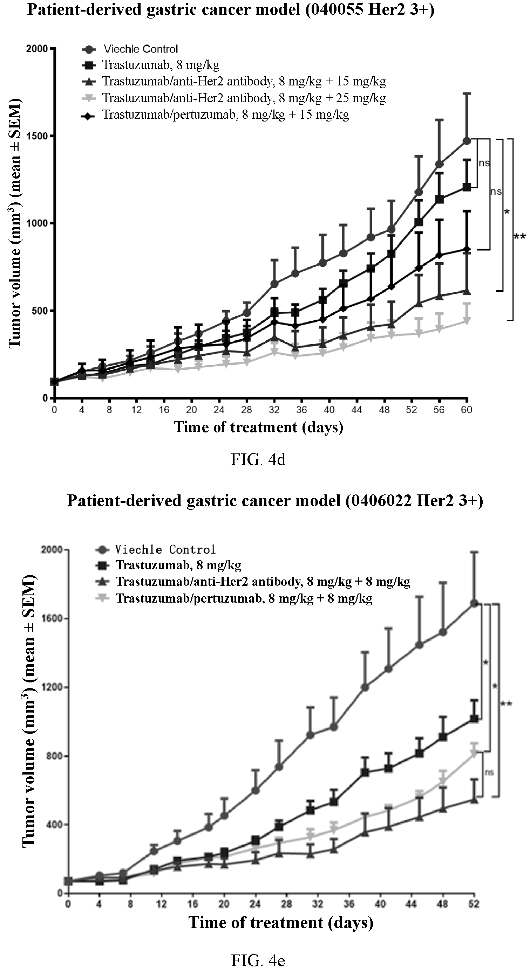

Referring to FIG. 4a, in the *in vitro* growth inhibition study of the NCI-N87 gastric cancer cells by the second anti-HER2 antibody or trastuzumab alone or in combination, the combination of trastuzumab and the second anti-HER2 antibody significantly enhanced the anti-proliferative activity of individual antibodies, with a better cell proliferation effect than that of the combination of trastuzumab and pertuzumab. Referring to FIG. 4b, according to the caspases 3/7 activity assay, the treatment of gastric cancer cells NCI-N87 with the combination of the second anti-HER2 antibody and trastuzumab induced the apoptosis of NCI-N87 cells, while the treatment of NCI-N87 gastric cancer cells with trastuzumab, the second anti-HER2 antibody, or pertuzumab alone, or the combination of trastuzumab and pertuzumab did not significantly induce the apoptosis of NCI-N87 cells. In the xenograft human gastric cancer NCI-N87 model treatment study shown in FIG. 4c, the combination of trastuzumab with the second anti-HER2 antibody significantly inhibited the size of NCI-N87 tumors in the model. In the PDX model treatment study shown in FIGs. 4d and 4e, the combination of trastuzumab and the second anti-HER2 antibody inhibited the tumor size.

In the above studies on the human gastric cancer cells, the xenograft human gastric cancer NCI-N87 model, and the PDX model, the combined use of trastuzumab and the second anti-HER2 antibody has a strong anti-tumor effect (on gastric cancer cells) to inhibit tumor growth and promote tumor regression. The enhanced anti-tumor effect of the combination suggests a unique synergistic effect of the combination of trastuzumab and the second anti-HER2 antibody. This is in part attributed to the ability of the anti-HER2 antibody and trastuzumab to induce the apoptosis of gastric cancer cells, which, however, was absent in studies where trastuzumab, the second anti-HER2 antibody, pertuzumab, and the combination of trastuzumab and pertuzumab were used to treat the gastric cancer cells.

**Example 4**. Clinical study of combination therapy with trastuzumab, second anti-HER2 antibody, and chemotherapeutic agent in patients with advanced unresectable or metastatic HER2-positive gastric cancer or gastroesophageal junction adenocarcinoma

This study is a randomized, double-blind, multi-site, phase II clinical study to evaluate the clinical efficacy and safety of trastuzumab + second anti-HER2 antibody + chemotherapeutic agent compared to placebo and trastuzumab + chemotherapeutic agent first-line treatment regimens in the treatment of patients with advanced unresectable or metastatic gastric cancer.

### Study Design

This study was a randomized, double-blind study. About 53 patients diagnosed with advanced unresectable or metastatic gastric cancer (untreated) were randomly divided into three groups in a ratio of about 1:1:1, and the corresponding treatment regimens are as follows:
Group A (treatment group): the second anti-HER2 antibody (25 mg/kg) + trastuzumab + chemotherapeutic agent (XELOX);
Group B (treatment group): the second anti-HER2 antibody (15 mg/kg) + trastuzumab + chemotherapeutic agent (XELOX);
Group C (standard treatment group): placebo (subjects in the unblinded and open-label part did not receive placebo) + trastuzumab + chemotherapeutic agent (XELOX).
The randomization program adopted a central random system and an interactive web response system (IWRS), the random stratification was performed according to the immunohistochemically (IHC/FISH) confirmed HER2 expression level (3+ or 2+), and the random codes were generated in a ratio of 1:1:1.

The modes of administration are as follows:
Second anti-HER2 antibody: 25 mg/kg or 15 mg/kg, intravenous infusion (IV), administered once every three weeks on the first day of each cycle.

Placebo: the same as the mode for administering the second anti-HER2 antibody (the dose varied according to the stage).

Trastuzumab: an initial loading dose of 8 mg/kg, followed by 6 mg/kg once every three weeks on the first day of each cycle; the time of the first infusion should be more than 90 minutes (inclusive), with 6 mg/kg repeated.

Oxaliplatin: 130 mg/m², IV infusion within 2-6 h (after the end of trastuzumab infusion), once every three weeks on the first day of each cycle for up to 8 cycles; if the body surface area exceeded 2.0 m², the investigator should calculate the dose based on 2.0 m² in clinical practice. Capecitabine: administered orally at 1000 mg/m² twice daily in cycles of three weeks from days 1 to 14 in each cycle; if the calculated theoretical dose exceeded 1500 mg, the investigator could select the dose of capecitabine at 1500 mg orally twice daily in clinical practice.

The patient tumor assessments were performed until investigator-decided termination after progression as per RECIST v1.1 criteria, loss of clinical benefits, intolerable toxicities, the patient- or investigator-decided treatment termination (abnormal treatment termination), deaths, withdrawal of informed consent, pregnancy, non-compliance with the protocol or procedure requirements, or administrative reasons (whichever comes first).

### Patient

Patients who were previously untreated and diagnosed with advanced unresectable or metastatic gastric cancer and met the criteria were enrolled in this study.

### Inclusion Criteria

1) Voluntary participation; complete understanding and knowledge of the study and written informed consent; willingness and ability to complete all study procedures;
2) Male or female, aged ≥ 18 years and ≤ 80 years upon informed consent;
3) histopathologically confirmed, unresectable HER2-positive locally advanced/metastatic gastric cancer or gastroesophageal junction adenocarcinoma (histopathologically confirmed adenocarcinoma);
4) Previously untreated with systemic anti-tumor therapies for locally advanced/metastatic gastric cancer or gastroesophageal junction adenocarcinoma;
5) For subjects who had previously received a neoadjuvant/adjuvant therapy, ≥ 6 months from the end of the last dose of previous treatment to the first dose of the study treatment;
6) For subjects who had previously received an anti-tumor traditional Chinese medicine or Chinese patent medicine treatment, the last dose should be prior to the randomization;
7) Treatment-related AEs returned to NCI-CTCAE grade ≤ 1 (except for alopecia);
8) At least one measurable lesion by central imaging evaluation according to Response Evaluation Criteria in Solid Tumors (RECIST) v1.1; measurable lesions should not have received local treatments such as radiotherapy (lesions in the irradiated area with confirmed progression may also be selected as target lesions); the target lesions should not be merely bone metastatic lesions;
9) HER2-positive tumors confirmed by HER2 expression level test in tumor tissues provided by the patient:
a. at least immunohistochemically (IHC)-confirmed HER2-positive 3+ (+++), or
b. at least IHC-confirmed HER2-positive 2+ (++) and fluorescence *in situ* hybridization (FISH)-confirmed positive;

10) ECOG score 0-1 within 7 days before randomization;
11) Expected survival ≥ 6 months;
12) hepatitis B surface antigen (HBsAg) (-) and hepatitis B core antibody (HBcAb) (-). If HBsAg (+) or HBcAb (+), the hepatitis B virus deoxyribonucleic acid (HBV-DNA) must be < 2500 copies/mL or 500 IU/mL or within the normal range of the study site.
13) HCV antibody (-); if HCV antibody (+), the HCV-RNA test must be negative for enrollment. Subjects with hepatitis B and hepatitis C co-infections were excluded (positive HBsAg or HBcAb and positive HCV antibody).
14) Normal main organ functions meeting the following criteria (no blood transfusion, or albumin, recombinant human thrombopoietin, or colony-stimulating factor (CSF) therapy within 14 days prior to the first dose of the study treatment):

**Table 3. Main organ function criteria**

| **Blood system** | |
|---|---|
| Absolute neutrophil count (ANC) | ≥ 1.5×10⁹/L |
| Platelet (PLT) | ≥ 100×10⁹/L |
| Hemoglobin (Hb) | ≥ 90g/L |

| **Liver function** | |
|---|---|
| Total bilirubin (TBIL) | ≤ 1.5 × upper limit of normal (ULN) |
| Alanine aminotransferase (ALT) | ≤ 2.5 × ULN; for patients with liver metastasis, ≤ 5.0 × ULN |
| Aspartate aminotransferase (AST) | ≤ 2.5 × ULN; for patients with liver metastasis, ≤ 5.0 × ULN |
| Alkaline phosphatase (ALP) | ≤ 2.5 × ULN; for patients with liver metastasis and/or bone metastasis, ≤ 5.0 × ULN |
| Albumin | ≥ 25 g/L |

| **Renal function** | |
|---|---|
| Creatinine (Cr) | ≤ 1.5 × ULN; if > 1.5 × ULN, the creatinine clearance rate should be ≥ 60 mL/min (calculated according to the Cockcroft-Gault formula) |

| **Coagulation** | |
|---|---|
| Activated partial thromboplastin time (APTT) | ≤ 1.5×ULN |
| Prothrombin time (PT) | ≤ 1.5×ULN |
| International normalized ratio (INR) | ≤ 1.5×ULN |

15) Female subjects of childbearing potential must have a negative blood pregnancy test within 7 days prior to the randomization. Female subjects of childbearing potential and male subjects whose partners were women of childbearing potential were required to take at least one medically accepted contraceptive measure (such as intrauterine devices, contraceptives, or condoms) during the study treatment period and for at least 6 months after the last dose of the second anti-HER2 antibody/trastuzumab/placebo and chemotherapeutic agent.

### Exclusion Criteria

Patients meeting any of the following exclusion criteria should be excluded from this study:
1) Other malignancies within 2 years before the first dose of the study treatment. Patients with localized cancers that have been cured, such as basal cell carcinoma, squamous cell carcinoma, superficial bladder cancer, prostate carcinoma *in situ*, cervical carcinoma *in situ*, and breast cancer *in situ* may be enrolled;
2) Disease progression within 6 months after neoadjuvant or adjuvant chemotherapy (or both) or radiotherapy for gastric adenocarcinoma or gastroesophageal junction adenocarcinoma;
3) Previous treatment with any therapy targeting HER2 or an immune checkpoint (e.g., PD-1, PD-L1, or CTLA-4);
4) Previous use of doxorubicin with an *in vivo* concentration > 360 mg/m² (or equivalent); note: equivalent therapies include epirubicin > 720 mg/m², mitoxantrone > 120 mg/m², idarubicin > 90 mg/m², doxorubicin liposomes with doxorubicin equivalent > 360 mg/m², or other anthracyclines. If more than one anthracycline is used, the cumulative dose must not exceed 360 mg/m² doxorubicin equivalent;
5) Uncontrolled pleural effusion or pericardial effusion after proper intervention, or ascites requiring frequent drainage;
6) Active bleeding in the gastrointestinal tract requiring blood transfusion therapy or invasive intervention;
7) Central nervous system (CNS) or leptomeningeal metastases;
8) Cerebrovascular accident, myocardial infarction, unstable angina pectoris, or poorly controlled arrhythmia (including QTc interval ≥ 450 ms for males and ≥ 470 ms for females, calculated according to the Fridericia formula) within half a year from the first dose;
9) Cardiac insufficiency of grade III or IV as per the New York Heart Association (NYHA) criteria, or an LVEF (left ventricular ejection fraction) < 55% by echocardiography;
10) Active tuberculosis;
11) Patients with previous or current interstitial pneumonia, pneumoconiosis, radiation-induced pneumonitis, drug-induced pneumonitis, severe lung function impairment or other conditions that may interfere with the detection and management of suspected drug-related pulmonary toxicities;
12) Treatment with live-attenuated vaccines within 28 days prior to the randomization, with the exception of inactivated virus vaccines for seasonal influenza or COVID-19;
13) Baseline chest imaging examination suggesting active lung inflammation accompanied by clinically relevant symptoms or signs;
14) Patients requiring systemic corticosteroid treatment (> 10 mg/day therapeutic dose of prednisone) or other immunosuppressive drugs within 14 days before the randomization or during the study. However, the following conditions were allowed for enrollment: in the absence of active autoimmune disease, patients were allowed to use topical or inhaled steroids or adrenal hormone replacement therapy ≤ 10 mg/day therapeutic dose of prednisone;
15) A major surgery within 28 days prior to the first dose of the study treatment, where the major surgery in this study is defined as: a surgery that requires at least 3 weeks of recovery before receiving the study treatment;
16) Radical radiotherapy within 3 months prior to the start of study treatment; palliative radiotherapy for bone disorders and superficial lesions that ended 14 days prior to the first dose was allowed; radiotherapy covering more than 30% of the bone marrow area within 28 days prior to the first dose was prohibited;
17) Current participation in another clinical study, or within 14 days from the end of treatment in the previous clinical study to the planned start of treatment in this study;
18) Known history of severe allergy to any monoclonal antibody or study drug excipients;
19) Evident pneumoperitoneum that cannot be explained by puncture or recent surgery;
20) Known history of psychotropic drug abuse or drug abuse;
21) Pregnant or lactating women;
22) Other factors, as determined by the investigator, that may result in premature discontinuation of treatment. For example, other serious medical conditions (including mental illnesses) requiring concomitant treatment, serious laboratory abnormalities, family or social factors, and other conditions that may affect subject safety or the collection of trial data.

### Results

The primary efficacy endpoints for this study were progression-free survival (PFS) and objective response rate (ORR). The progression-free survival (PFS) is defined as the time (in months) from the randomization to the first confirmed or recorded (IRRC-assessed) disease progression or death (whichever comes first) as per RECIST v1.1 criteria. For subjects without disease progression or death at the time of analysis, the date of their last objective tumor assessment is the censored date of PFS. The objective response rate (ORR) is defined as the proportion of subjects who achieved (investigator- and IRRC-) assessed CR and PR as the optimal response as per RECIST v1.1 criteria. The objective response rates of the groups were analyzed descriptively, and the corresponding 95% confidence interval was given. The median progression-free survival was estimated using the Kaplan-Meier method, and Kaplan-Meier curves were plotted. The HR and its 95% confidence interval for PFS were estimated by a stratified COX proportional hazards model. The PFS was compared between the groups using a stratified log-rank test. The ORR difference was tested between the groups using a stratified Cochran-Mantel-Haenszel (CMH) method to estimate the odds ratio and its 95% confidence interval.

The secondary efficacy endpoint in this study was overall survival, and the statistical methods for the investigator-assessed progression-free survival and objective response rate as per RECIST v1.1 criteria were the same as those for the primary efficacy endpoints. The duration of response refers to the time from the date of the first achieved CR or PR (whichever comes first) to the date of first recorded disease progression or death (whichever comes first). The DOR was evaluated for subjects with investigator- and IRRC-assessed objective response. The median was estimated using the Kaplan-Meier method, and Kaplan-Meier curves were plotted.

### Efficacy

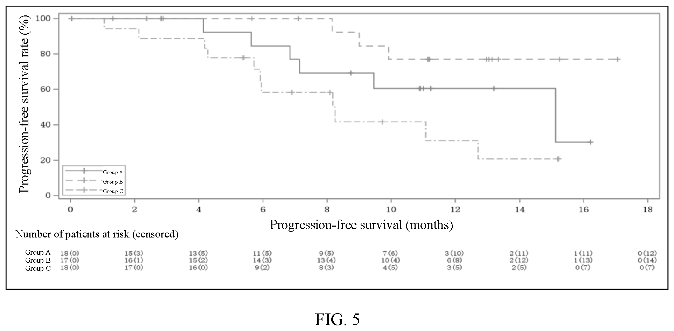

A total of 53 patients were randomized into groups A, B, and C and included in the analysis, with 18 in group A, 17 in group B, and 18 in group C. The median progression-free survival of the 53 subjects as assessed by imaging was as follows: the PFS of the patients in group A was 15.1 months (95% confidence interval: 6.8 months - unevaluable), the PFS of the patients in group B was NA (95% confidence interval: 9.9 months - unevaluable), and the PFS of the patients in group C was 8.2 months (95% confidence interval: 9.9 months - unevaluable). In this analysis, a total of 12 (28.3%) deaths were reported. The overall survival data were not mature, and the median overall survival time was not reached in all groups. The K-M curves for the progression-free survival of the subjects are shown in FIG. 5, and the summary of efficacy data of the patients is shown in Table 4.

**Table 4. Summary of efficacy data**

| | **Group A (n=18)** | **Group B (n=17)** | **Group C (n=18)** |
|---|---|---|---|
| Median progression-free survival (months, 95% CI) | 15.11(6.83, -) | NR(9.92, -) | 8.21(5.72,12.71) |
| Hazard ratio (95% CI) (vs. group C) | 0.5 (0.17,1.27) | 0.1 (0.04,0.52) | / |
| | p = 0.1272 (descriptive) | p = 0.0007 (descriptive) | |
| Number of progression-free survival events | 6 | 3 | 11 |
| Median duration of response (months, 95% CI) | 12.4(5.49, -) | NR(8.57,-) | 6.8(4.37,-) |
| Hazard ratio (95% CI) (vs. group C) | 0.6 (0.20,1.62) | 0.1 (0.02,0.50) | |
| | p = 0.2848 (descriptive) | p = 0.0006 (descriptive) | |
| Confirmed objective response rate | 77.8% | 82.4% | 88.9% |
| 18-week objective response rate | 72.2% | 82.4% | 66.7% |
| 36-week objective response rate | 44.4% | 64.7% | 27.8% |
| 48-week objective response rate | 38.9% | 58.8% | 16.7% |
| Median overall survival (months, 95% CI) | NR(12.42, -) | NR(-, -) | NR(6.44,-) |
| Hazard ratio (95% CI) (vs. group C) | 0.4 (0.13,1.45) | 0.3 (0.09,1.26) | / |
| | p = 0.1621 (descriptive) | p = 0.0894 (descriptive) | |
| Median follow-up time (months) | 13.16 | 14.16 | 12.91 |

Referring to Table 4, for group A, the median progression-free survival was 15.11 months, and the median duration of response was 12.4 months; for group B, the median progression-free survival and the median duration of response were NA; for group C, the median progression-free survival was 8.21 months and the median duration of response was 6.8 months. That is, compared with the combination of trastuzumab + the chemotherapeutic agent, the combination of trastuzumab + the second anti-HER2 antibody + the chemotherapeutic agent (groups A and B) can significantly prolong the median progression-free survival and the median duration of response in patients. The hazard ratio of median progression-free survival was 0.5 (95% CI, 0.17-1.27, P = 0.1272) for group A and 0.1 (95% CI, 0.04-0.52, P = 0.0007) for group B, and the hazard ratio of median duration of response was 0.6 (95% CI, 0.20-1.62, P = 0.2848) for group A and 0.1 (95% CI, 0.02-0.50, P = 0.0006) for group B, suggesting the superiority of the trastuzumab + second anti-HER2 antibody + chemotherapeutic agent combination groups.

The objective response rates of the two treatment groups (group A and group B) were significantly improved compared with the control group (group C), where the 36-week objective response rates of group A and group B were 44.4% and 64.7%, respectively, and the 48-week objective response rates of group A and group B were 38.9% and 58.8%, respectively.

In the treatment of patients with HER2-positive advanced gastric cancer in this study, the combination therapy of the second anti-HER2 antibody and the standard therapy (trastuzumab + XELOX) exhibited significant and durable response and provided patients with a significantly prolonged progression-free survival.

### Safety

In the phase I clinical trial of the second anti-HER2 antibody, as of April 22, 2022, a total of 11 subjects were enrolled and given at least one dose of the anti-HER2 antibody. The 11 subjects were included in the safety analysis set. The results show that the second anti-HER2 antibody was well tolerated in all treatment groups. No dose-related toxic reactions were observed, and no treatment-emergent adverse events leading to interruption, dose reduction, or infusion rate reduction occurred. Treatment-emergent adverse events included decreased lymphocyte count, decreased leukocyte count, and hypokalemia.

In this study, as of July 30, 2023, a total of 53 subjects were enrolled and given at least one dose of trastuzumab + the second anti-HER2 antibody + a chemotherapeutic agent. The 53 subjects were included in the safety analysis set. The most common treatment-emergent adverse events (TEAEs) were decreased platelet count (group A vs group B vs group C, 55.6% vs 76.5% vs 83.3%), anemia (66.7% vs 58.8% vs 72.2%), decreased neutrophil count (72.2% vs 64.7% vs 55.6%), and decreased leukocyte count (72.2% vs 52.9% vs 61.1%). Grade 3 or higher treatment-emergent adverse events in this study included 13 (72.2%) in group A, 7 (41.2%) in group B, and 8 (44.4%) in group C, among which the most common adverse events were decreased platelet count and decreased neutrophil count. The summary of subject efficacy data is shown in Table 5.

**Table 5. Summary of safety data**

| | **Group A (n=18)** | **Group B (n=17)** | **Group C (n=18)** |
|---|---|---|---|
| Treatment-emergent adverse event | 18 (100) | 16 (94.1) | 18 (100) |
| Grade 3 or higher treatment-emergent adverse event | 13 (72.2) | 7 (41.2) | 8 (44.4) |
| Serious treatment-emergent adverse event | 8 (44.4) | 4 (23.5) | 5 (27.8) |
| Treatment-emergent adverse events related to second anti-HER2 antibody/placebo | 17 (94.4) | 15 (82.2) | 11 (61.1) |
| Grade 3 or higher treatment-emergent adverse event related to second anti-HER2 antibody/placebo | 10 (56.6) | 3 (17.6) | 3 (16.7) |
| Serious treatment-emergent adverse event related to second anti-HER2 antibody/placebo | 5 (27.8) | 1 (5.9) | 1 (5.6) |
| Treatment-emergent adverse events related to second anti-HER2 antibody/placebo leading to discontinuation of study treatment | 1 (5.6) | 1 (5.9) | 1 (5.6) |
| Treatment-emergent adverse events related to second anti-HER2 antibody/placebo leading to death | 0 | 0 | 1 (5.6) |

The safety data of the treatment groups (groups A and B), especially group B, of the study were substantially consistent with that of the standard treatment group (group C, trastuzumab + XELOX). No subject in the treatment groups experienced death related to the second anti-HER2 antibody, indicating good safety and tolerability.

### Conclusion

For the first-line standard treatment (trastuzumab + a chemotherapeutic agent) for HER2-positive advanced gastric cancer, the median overall survival was 13.8 months, and the median progression-free survival was 6.7 months. In this study, the combination therapy of the second anti-HER2 antibody with the standard therapy (trastuzumab + a chemotherapeutic agent) shows a significant therapeutic advantage over the current standard treatment: the median progression-free survival of the high-dose group (group A) reached 15.1 months, and the median overall survival had exceeded the registered historical data, while the median progression-free survival of the low-dose group (group B) was still not reached and was expected to exceed that of group A, which had a hazard ratio of 0.71 (95% confidence interval: 0.04, 0.52) and a p value of 0.0007 as compared with the standard treatment group (group C). Therefore, the combination therapy of the second anti-HER2 antibody + trastuzumab + a chemotherapeutic agent involved in the present disclosure shows an efficacy advantage significantly superior to that of the standard treatment, as well as good safety and tolerability.

It will be appreciated that various modifications or changes may be made by those skilled in the art after reading the aforementioned content of the present disclosure, and such equivalent forms also fall within the scope defined by the claims of the present application.

## Claims

1. A method for treating HER2-positive gastric cancer or gastroesophageal junction adenocarcinoma in a patient, comprising administering to the patient a therapeutically effective amount of:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

2. The method according to claim 1, wherein the chemotherapeutic agent is a fluorouracil drug.

3. The method according to claim 1, wherein the chemotherapeutic agent is a platinum-based drug.

4. The method according to any one of claims 1-3, wherein the chemotherapeutic agent is a combination of a fluorouracil drug and a platinum-based drug.

5. The method according to any one of claims 2-4, wherein the fluorouracil drug is selected from 5-fluorouracil and capecitabine.

6. The method according to any one of claims 3-4, wherein the platinum-based drug is selected from cisplatin and oxaliplatin.

7. The method according to any one of claims 2-6, wherein the fluorouracil drug is capecitabine.

8. The method according to any one of claims 3-6, wherein the platinum-based drug is oxaliplatin.

9. The method according to any one of claims 1-8, wherein the chemotherapeutic agent is a combination of capecitabine and oxaliplatin.

10. The method according to any one of claims 1-9, wherein the cancer is HER2-positive advanced gastric cancer.

11. The method according to any one of claims 1-10, wherein the cancer is unresectable or metastatic HER2-positive gastric cancer or gastroesophageal junction adenocarcinoma.

12. The method according to any one of claims 1-11, wherein the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

13. The method according to any one of claims 1-12, wherein the heavy chain of the second antibody comprises or consists of the amino acid sequence set forth in SEQ ID NO: 5, and the light chain of the second antibody comprises or consists of the amino acid sequence set forth in SEQ ID NO: 10.

14. The method according to any one of claims 1-13, wherein trastuzumab is administered at a body weight-based dose of about 0.1 mg/kg to about 10 mg/kg.

15. The method according to any one of claims 1-14, wherein trastuzumab is administered at a body weight-based dose of about 0.1 mg/kg to about 10 mg/kg, about 0.3 mg/kg to about 10 mg/kg, 0.9 mg/kg to about 10 mg/kg, about 1 mg/kg to about 10 mg/kg, about 2.5 mg/kg to about 10 mg/kg, about 3 mg/kg to about 10 mg/kg, about 4 mg/kg to about 10 mg/kg, about 5 mg/kg to about 10 mg/kg, about 6 mg/kg to about 10 mg/kg, about 7 mg/kg to about 10 mg/kg, about 8 mg/kg to about 10 mg/kg, about 9 mg/kg to about 10 mg/kg, about 0.1 mg/kg to about 8 mg/kg, about 0.3 mg/kg to about 8 mg/kg, 0.9 mg/kg to about 8 mg/kg, about 1 mg/kg to about 8 mg/kg, about 2.5 mg/kg to about 8 mg/kg, about 3 mg/kg to about 8 mg/kg, about 4 mg/kg to about 8 mg/kg, about 5 mg/kg to about 8 mg/kg, about 6 mg/kg to about 8 mg/kg, about 7 mg/kg to about 8 mg/kg, about 0.1 mg/kg to about 6 mg/kg, about 0.3 mg/kg to about 6 mg/kg, 0.9 mg/kg to about 6 mg/kg, about 1 mg/kg to about 6 mg/kg, about 2.5 mg/kg to about 6 mg/kg, about 3 mg/kg to about 6 mg/kg, about 4 mg/kg to about 6 mg/kg, or about 5 mg/kg to about 6 mg/kg.

16. The method according to any one of claims 1-15, wherein trastuzumab is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

17. The method according to any one of claims 1-16, wherein on a body weight basis, trastuzumab is administered at an initial loading dose of about 8 mg/kg followed by a dose of about 6 mg/kg.

18. The method according to any one of claims 1-17, wherein trastuzumab is administered about once every week, about once every two weeks, about once every three weeks, about once every four weeks, about once every month, about once every 3-6 months, or longer.

19. The method according to any one of claims 1-18, wherein trastuzumab is administered intravenously.

20. The method according to any one of claims 1-19, wherein the second antibody is administered at a body weight-based dose of about 0.1 mg/kg to about 30 mg/kg, about 0.3 mg/kg to about 30 mg/kg, 0.9 mg/kg to about 30 mg/kg, about 1 mg/kg to about 30 mg/kg, about 2.5 mg/kg to about 30 mg/kg, about 3 mg/kg to about 30 mg/kg, about 4 mg/kg to about 30 mg/kg, about 5 mg/kg to about 30 mg/kg, about 6 mg/kg to about 30 mg/kg, about 7 mg/kg to about 30 mg/kg, about 8 mg/kg to about 30 mg/kg, about 9 mg/kg to about 30 mg/kg, about 10 mg/kg to about 30 mg/kg, about 11 mg/kg to about 30 mg/kg, about 12 mg/kg to about 30 mg/kg, about 13 mg/kg to about 30 mg/kg, about 14 mg/kg to about 30 mg/kg, about 15 mg/kg to about 30 mg/kg, about 16 mg/kg to about 30 mg/kg, about 17 mg/kg to about 30 mg/kg, about 18 mg/kg to about 30 mg/kg, about 19 mg/kg to about 30 mg/kg, about 20 mg/kg to about 30 mg/kg, about 21 mg/kg to about 30 mg/kg, about 22 mg/kg to about 30 mg/kg, about 23 mg/kg to about 30 mg/kg, about 24 mg/kg to about 30 mg/kg, about 25 mg/kg to about 30 mg/kg, about 26 mg/kg to about 30 mg/kg, about 27 mg/kg to about 30 mg/kg, about 28 mg/kg to about 30 mg/kg, about 29 mg/kg to about 30 mg/kg, about 0.1 mg/kg to about 25 mg/kg, about 0.3 mg/kg to about 25 mg/kg, 0.9 mg/kg to about 25 mg/kg, about 1 mg/kg to about 25 mg/kg, about 2.5 mg/kg to about 25 mg/kg, about 3 mg/kg to about 25 mg/kg, about 4 mg/kg to about 25 mg/kg, about 5 mg/kg to about 25 mg/kg, about 6 mg/kg to about 25 mg/kg, about 7 mg/kg to about 25 mg/kg, about 8 mg/kg to about 25 mg/kg, about 9 mg/kg to about 25 mg/kg, about 10 mg/kg to about 25 mg/kg, about 11 mg/kg to about 25 mg/kg, about 12 mg/kg to about 25 mg/kg, about 13 mg/kg to about 25 mg/kg, about 14 mg/kg to about 25 mg/kg, about 15 mg/kg to about 25 mg/kg, about 16 mg/kg to about 25 mg/kg, about 17 mg/kg to about 25 mg/kg, about 18 mg/kg to about 25 mg/kg, about 19 mg/kg to about 25 mg/kg, about 20 mg/kg to about 25 mg/kg, about 21 mg/kg to about 25 mg/kg, about 22 mg/kg to about 25 mg/kg, about 23 mg/kg to about 25 mg/kg, about 24 mg/kg to about 25 mg/kg, about 0.1 mg/kg to about 20 mg/kg, about 0.3 mg/kg to about 20 mg/kg, 0.9 mg/kg to about 20 mg/kg, about 1 mg/kg to about 20 mg/kg, about 2.5 mg/kg to about 20 mg/kg, about 3 mg/kg to about 20 mg/kg, about 4 mg/kg to about 20 mg/kg, about 5 mg/kg to about 20 mg/kg, about 6 mg/kg to about 20 mg/kg, about 7 mg/kg to about 20 mg/kg, about 8 mg/kg to about 20 mg/kg, about 9 mg/kg to about 20 mg/kg, about 10 mg/kg to about 20 mg/kg, about 11 mg/kg to about 20 mg/kg, about 12 mg/kg to about 20 mg/kg, about 13 mg/kg to about 20 mg/kg, about 14 mg/kg to about 20 mg/kg, about 15 mg/kg to about 20 mg/kg, about 16 mg/kg to about 20 mg/kg, about 17 mg/kg to about 20 mg/kg, about 18 mg/kg to about 20 mg/kg, about 19 mg/kg to about 20 mg/kg, about 0.1 mg/kg to about 15 mg/kg, about 0.3 mg/kg to about 15 mg/kg, 0.9 mg/kg to about 15 mg/kg, about 1 mg/kg to about 15 mg/kg, about 2.5 mg/kg to about 15 mg/kg, about 3 mg/kg to about 15 mg/kg, about 4 mg/kg to about 15 mg/kg, about 5 mg/kg to about 15 mg/kg, about 6 mg/kg to about 15 mg/kg, about 7 mg/kg to about 15 mg/kg, about 8 mg/kg to about 15 mg/kg, about 9 mg/kg to about 15 mg/kg, about 10 mg/kg to about 15 mg/kg, about 11 mg/kg to about 15 mg/kg, about 12 mg/kg to about 15 mg/kg, about 13 mg/kg to about 15 mg/kg, or about 14 mg/kg to about 15 mg/kg.

21. The method according to any one of claims 1-20, wherein the second antibody is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, or about 30 mg/kg.

22. The method according to any one of claims 1-21, wherein the second antibody is administered at a body weight-based dose of about 25 mg/kg or 15 mg/kg.

23. The method according to any one of claims 1-22, wherein the second antibody is administered about once every week, about once every two weeks, about once every three weeks, about once every four weeks, about once every month, about once every 3-6 months, or longer.

24. The method according to any one of claims 1-23, wherein the second antibody is administered intravenously.

25. The method according to any one of claims 6-24, wherein oxaliplatin is administered at a body surface area-based dose of about 10 to about 150 mg/m², about 20 to about 150 mg/m², about 30 to about 150 mg/m², about 40 to about 150 mg/m², about 50 to about 150 mg/m², about 60 to about 150 mg/m², about 70 to about 150 mg/m², about 75 to about 150 mg/m², about 80 to about 150 mg/m², about 85 to about 150 mg/m², about 90 to about 150 mg/m², about 95 to about 150 mg/m², about 100 to about 150 mg/m², about 105 to about 150 mg/m², about 110 to about 150 mg/m², about 115 to about 150 mg/m², about 120 to about 150 mg/m², about 125 to about 150 mg/m², about 130 to about 150 mg/m², about 135 to about 150 mg/m², about 140 to about 150 mg/m², about 145 to about 150 mg/m², about 10 to about 140 mg/m², about 20 to about 140 mg/m², about 30 to about 140 mg/m², about 40 to about 140 mg/m², about 50 to about 140 mg/m², about 60 to about 140 mg/m², about 70 to about 140 mg/m², about 75 to about 140 mg/m², about 80 to about 140 mg/m², about 85 to about 140 mg/m², about 90 to about 140 mg/m², about 95 to about 140 mg/m², about 100 to about 140 mg/m², about 105 to about 140 mg/m², about 110 to about 140 mg/m², about 115 to about 140 mg/m², about 120 to about 140 mg/m², about 125 to about 140 mg/m², about 130 to about 140 mg/m², about 135 to about 140 mg/m², about 10 to about 130 mg/m², about 20 to about 130 mg/m², about 30 to about 130 mg/m², about 40 to about 130 mg/m², about 50 to about 130 mg/m², about 60 to about 130 mg/m², about 70 to about 130 mg/m², about 75 to about 130 mg/m², about 80 to about 130 mg/m², about 85 to about 130 mg/m², about 90 to about 130 mg/m², about 95 to about 130 mg/m², about 100 to about 130 mg/m², about 105 to about 130 mg/m², about 110 to about 130 mg/m², about 115 to about 130 mg/m², about 120 to about 130 mg/m², or about 125 to about 130 mg/m².

26. The method according to any one of claims 6-25, wherein oxaliplatin is administered at a body surface area-based dose of about 10 mg/m², about 20 mg/m², about 30 mg/m², about 40 mg/m², about 50 mg/m², about 60 mg/m², about 70 mg/m², about 75 mg/m², about 80 mg/m², about 85 mg/m², about 90 mg/m², about 95 mg/m², about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², or about 150 mg/m².

27. The method according to any one of claims 6-26, wherein oxaliplatin is administered at a body surface area-based dose of about 130 mg/m².

28. The method according to any one of claims 6-27, wherein oxaliplatin is administered about once every week, about once every two weeks, about once every three weeks, about once every four weeks, about once every month, about once every 3-6 months, or longer.

29. The method according to any one of claims 6-28, wherein oxaliplatin is administered intravenously.

30. The method according to any one of claims 7-29, wherein capecitabine is administered at a body surface area-based dose of about 100 to about 1200 mg/m², about 200 to about 1200 mg/m², about 300 to about 1200 mg/m², about 400 to about 1200 mg/m², about 500 to about 1200 mg/m², about 600 to about 1200 mg/m², about 650 to about 1200 mg/m², about 700 to about 1200 mg/m², about 750 to about 1200 mg/m², about 800 to about 1200 mg/m², about 850 to about 1200 mg/m², about 900 to about 1200 mg/m², about 950 to about 1200 mg/m², about 1000 to about 1200 mg/m², about 1050 to about 1200 mg/m², about 1100 to about 1200 mg/m², about 1150 to about 1200 mg/m², about 100 to about 1100 mg/m², about 200 to about 1100 mg/m², about 300 to about 1100 mg/m², about 400 to about 1100 mg/m², about 500 to about 1100 mg/m², about 600 to about 1100 mg/m², about 650 to about 1100 mg/m², about 700 to about 1100 mg/m², about 750 to about 1100 mg/m², about 800 to about 1100 mg/m², about 850 to about 1100 mg/m², about 900 to about 1100 mg/m², about 950 to about 1100 mg/m², about 1000 to about 1100 mg/m², about 100 to about 1000 mg/m², about 200 to about 1000 mg/m², about 300 to about 1000 mg/m², about 400 to about 1000 mg/m², about 500 to about 1000 mg/m², about 600 to about 1000 mg/m², about 650 to about 1000 mg/m², about 700 to about 1000 mg/m², about 750 to about 1000 mg/m², about 800 to about 1000 mg/m², about 850 to about 1000 mg/m², about 900 to about 1000 mg/m², or about 950 to about 1000 mg/m².

31. The method according to any one of claims 7-30, wherein capecitabine is administered at a body surface area-based dose of about 100 mg/m², about 200 mg/m², about 300 mg/m², about 400 mg/m², about 500 mg/m², about 600 mg/m², about 650 mg/m², about 700 mg/m², about 750 mg/m², about 800 mg/m², about 850 mg/m², about 900 mg/m², about 950 mg/m², about 1000 mg/m², about 1050 mg/m², about 1100 mg/m², about 1150 mg/m², or about 1200 mg/m².

32. The method according to any one of claims 7-31, wherein capecitabine is administered at a body surface area-based dose of about 1000 mg/m².

33. The method according to any one of claims 7-32, wherein capecitabine is administered once daily, twice daily, or thrice daily.

34. The method according to any one of claims 7-33, wherein capecitabine is administered orally.

35. The method according to any one of claims 1-34, wherein on a body weight basis, trastuzumab is administered at an initial loading dose of about 8 mg/kg followed by a dose of about 6 mg/kg; the second antibody is administered at a body weight-based dose of about 25 mg/kg or 15 mg/kg; oxaliplatin is administered at a body surface area-based dose of about 130 mg/m²; capecitabine is administered at a body surface area-based dose of about 1000 mg/m².

36. The method according to any one of claims 1-35, wherein the second antibody is administered to the patient before the administration of trastuzumab.

37. The method according to any one of claims 1-36, wherein the second antibody is administered to the patient after the administration of trastuzumab.

38. The method according to any one of claims 1-37, wherein trastuzumab and the second antibody are administered concurrently.

39. The method according to any one of claims 1-38, further comprising administering an immune checkpoint inhibitor.

40. The method according to claim 39, wherein the immune checkpoint inhibitor is an anti-PD-1 antibody or an anti-PD-L1 antibody.

41. The method according to claim 39 or 40, wherein the immune checkpoint inhibitor is an anti-PD-1 antibody.

42. A combination therapy for treating HER2-positive gastric cancer or gastroesophageal junction adenocarcinoma in a patient, wherein the treatment comprises administering to the patient the combination therapy; the combination therapy comprises:
(1) trastuzumab;
(2) a second antibody, wherein the second antibody is a second anti-HER2 antibody or an antigen-binding fragment thereof having a different binding epitope from trastuzumab, comprising a heavy chain and a light chain, wherein the heavy chain comprises a heavy chain variable region comprising the HCDR1 set forth in SEQ ID NO: 1, the HCDR2 set forth in SEQ ID NO: 2, and the HCDR3 set forth in SEQ ID NO: 3, and the light chain comprises a light chain variable region comprising the LCDR1 set forth in SEQ ID NO: 6, the LCDR2 set forth in SEQ ID NO: 7, and the LCDR3 set forth in SEQ ID NO: 8; and
(3) a chemotherapeutic agent.

43. The combination therapy according to claim 42, wherein the chemotherapeutic agent is a fluorouracil drug.

44. The combination therapy according to claim 42, wherein the chemotherapeutic agent is a platinum-based drug.

45. The combination therapy according to any one of claims 42-44, wherein the chemotherapeutic agent is a combination of a fluorouracil drug and a platinum-based drug.

46. The combination therapy according to any one of claims 43-45, wherein the fluorouracil drug is selected from 5-fluorouracil and capecitabine.

47. The combination therapy according to any one of claims 44-46, wherein the platinum-based drug is selected from cisplatin and oxaliplatin.

48. The combination therapy according to any one of claims 43-47, wherein the fluorouracil drug is capecitabine.

49. The combination therapy according to any one of claims 44-47, wherein the platinum-based drug is oxaliplatin.

50. The combination therapy according to any one of claims 42-49, wherein the chemotherapeutic agent is a combination of capecitabine and oxaliplatin.

51. The combination therapy according to any one of claims 42-50, wherein the cancer is HER2-positive advanced gastric cancer.

52. The combination therapy according to any one of claims 42-51, wherein the cancer is unresectable or metastatic HER2-positive gastric cancer or gastroesophageal junction adenocarcinoma.

53. The combination therapy according to any one of claims 42-52, wherein the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

54. The combination therapy according to any one of claims 42-53, wherein the heavy chain of the second antibody comprises or consists of the amino acid sequence set forth in SEQ ID NO: 5, and the light chain of the second antibody comprises or consists of the amino acid sequence set forth in SEQ ID NO: 10.

55. The combination therapy according to any one of claims 42-54, wherein trastuzumab is administered at a body weight-based dose of about 0.1 mg/kg to about 10 mg/kg.

56. The combination therapy according to any one of claims 42-55, wherein trastuzumab is administered at a body weight-based dose of about 0.1 mg/kg to about 10 mg/kg, about 0.3 mg/kg to about 10 mg/kg, 0.9 mg/kg to about 10 mg/kg, about 1 mg/kg to about 10 mg/kg, about 2.5 mg/kg to about 10 mg/kg, about 3 mg/kg to about 10 mg/kg, about 4 mg/kg to about 10 mg/kg, about 5 mg/kg to about 10 mg/kg, about 6 mg/kg to about 10 mg/kg, about 7 mg/kg to about 10 mg/kg, about 8 mg/kg to about 10 mg/kg, about 9 mg/kg to about 10 mg/kg, about 0.1 mg/kg to about 8 mg/kg, about 0.3 mg/kg to about 8 mg/kg, 0.9 mg/kg to about 8 mg/kg, about 1 mg/kg to about 8 mg/kg, about 2.5 mg/kg to about 8 mg/kg, about 3 mg/kg to about 8 mg/kg, about 4 mg/kg to about 8 mg/kg, about 5 mg/kg to about 8 mg/kg, about 6 mg/kg to about 8 mg/kg, about 7 mg/kg to about 8 mg/kg, about 0.1 mg/kg to about 6 mg/kg, about 0.3 mg/kg to about 6 mg/kg, 0.9 mg/kg to about 6 mg/kg, about 1 mg/kg to about 6 mg/kg, about 2.5 mg/kg to about 6 mg/kg, about 3 mg/kg to about 6 mg/kg, about 4 mg/kg to about 6 mg/kg, or about 5 mg/kg to about 6 mg/kg.

57. The combination therapy according to any one of claims 42-56, wherein trastuzumab is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg.

58. The combination therapy according to any one of claims 42-57, wherein on a body weight basis, trastuzumab is administered at an initial loading dose of about 8 mg/kg followed by a dose of about 6 mg/kg.

59. The combination therapy according to any one of claims 42-58, wherein trastuzumab is administered about once every week, about once every two weeks, about once every three weeks, about once every four weeks, about once every month, about once every 3-6 months, or longer.

60. The combination therapy according to any one of claims 42-59, wherein trastuzumab is administered intravenously.

61. The combination therapy according to any one of claims 42-60, wherein the second antibody is administered at a body weight-based dose of about 0.1 mg/kg to about 30 mg/kg, about 0.3 mg/kg to about 30 mg/kg, 0.9 mg/kg to about 30 mg/kg, about 1 mg/kg to about 30 mg/kg, about 2.5 mg/kg to about 30 mg/kg, about 3 mg/kg to about 30 mg/kg, about 4 mg/kg to about 30 mg/kg, about 5 mg/kg to about 30 mg/kg, about 6 mg/kg to about 30 mg/kg, about 7 mg/kg to about 30 mg/kg, about 8 mg/kg to about 30 mg/kg, about 9 mg/kg to about 30 mg/kg, about 10 mg/kg to about 30 mg/kg, about 11 mg/kg to about 30 mg/kg, about 12 mg/kg to about 30 mg/kg, about 13 mg/kg to about 30 mg/kg, about 14 mg/kg to about 30 mg/kg, about 15 mg/kg to about 30 mg/kg, about 16 mg/kg to about 30 mg/kg, about 17 mg/kg to about 30 mg/kg, about 18 mg/kg to about 30 mg/kg, about 19 mg/kg to about 30 mg/kg, about 20 mg/kg to about 30 mg/kg, about 21 mg/kg to about 30 mg/kg, about 22 mg/kg to about 30 mg/kg, about 23 mg/kg to about 30 mg/kg, about 24 mg/kg to about 30 mg/kg, about 25 mg/kg to about 30 mg/kg, about 26 mg/kg to about 30 mg/kg, about 27 mg/kg to about 30 mg/kg, about 28 mg/kg to about 30 mg/kg, about 29 mg/kg to about 30 mg/kg, about 0.1 mg/kg to about 25 mg/kg, about 0.3 mg/kg to about 25 mg/kg, 0.9 mg/kg to about 25 mg/kg, about 1 mg/kg to about 25 mg/kg, about 2.5 mg/kg to about 25 mg/kg, about 3 mg/kg to about 25 mg/kg, about 4 mg/kg to about 25 mg/kg, about 5 mg/kg to about 25 mg/kg, about 6 mg/kg to about 25 mg/kg, about 7 mg/kg to about 25 mg/kg, about 8 mg/kg to about 25 mg/kg, about 9 mg/kg to about 25 mg/kg, about 10 mg/kg to about 25 mg/kg, about 11 mg/kg to about 25 mg/kg, about 12 mg/kg to about 25 mg/kg, about 13 mg/kg to about 25 mg/kg, about 14 mg/kg to about 25 mg/kg, about 15 mg/kg to about 25 mg/kg, about 16 mg/kg to about 25 mg/kg, about 17 mg/kg to about 25 mg/kg, about 18 mg/kg to about 25 mg/kg, about 19 mg/kg to about 25 mg/kg, about 20 mg/kg to about 25 mg/kg, about 21 mg/kg to about 25 mg/kg, about 22 mg/kg to about 25 mg/kg, about 23 mg/kg to about 25 mg/kg, about 24 mg/kg to about 25 mg/kg, about 0.1 mg/kg to about 20 mg/kg, about 0.3 mg/kg to about 20 mg/kg, 0.9 mg/kg to about 20 mg/kg, about 1 mg/kg to about 20 mg/kg, about 2.5 mg/kg to about 20 mg/kg, about 3 mg/kg to about 20 mg/kg, about 4 mg/kg to about 20 mg/kg, about 5 mg/kg to about 20 mg/kg, about 6 mg/kg to about 20 mg/kg, about 7 mg/kg to about 20 mg/kg, about 8 mg/kg to about 20 mg/kg, about 9 mg/kg to about 20 mg/kg, about 10 mg/kg to about 20 mg/kg, about 11 mg/kg to about 20 mg/kg, about 12 mg/kg to about 20 mg/kg, about 13 mg/kg to about 20 mg/kg, about 14 mg/kg to about 20 mg/kg, about 15 mg/kg to about 20 mg/kg, about 16 mg/kg to about 20 mg/kg, about 17 mg/kg to about 20 mg/kg, about 18 mg/kg to about 20 mg/kg, about 19 mg/kg to about 20 mg/kg, about 0.1 mg/kg to about 15 mg/kg, about 0.3 mg/kg to about 15 mg/kg, 0.9 mg/kg to about 15 mg/kg, about 1 mg/kg to about 15 mg/kg, about 2.5 mg/kg to about 15 mg/kg, about 3 mg/kg to about 15 mg/kg, about 4 mg/kg to about 15 mg/kg, about 5 mg/kg to about 15 mg/kg, about 6 mg/kg to about 15 mg/kg, about 7 mg/kg to about 15 mg/kg, about 8 mg/kg to about 15 mg/kg, about 9 mg/kg to about 15 mg/kg, about 10 mg/kg to about 15 mg/kg, about 11 mg/kg to about 15 mg/kg, about 12 mg/kg to about 15 mg/kg, about 13 mg/kg to about 15 mg/kg, or about 14 mg/kg to about 15 mg/kg.

62. The combination therapy according to any one of claims 42-61, wherein the second antibody is administered at a body weight-based dose of about 0.1 mg/kg, about 0.3 mg/kg, about 0.9 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, about 20 mg/kg, about 21 mg/kg, about 22 mg/kg, about 23 mg/kg, about 24 mg/kg, about 25 mg/kg, about 26 mg/kg, about 27 mg/kg, about 28 mg/kg, about 29 mg/kg, or about 30 mg/kg.

63. The combination therapy according to any one of claims 42-62, wherein the second antibody is administered at a body weight-based dose of about 25 mg/kg or 15 mg/kg.

64. The combination therapy according to any one of claims 42-63, wherein the second antibody is administered about once every week, about once every two weeks, about once every three weeks, about once every four weeks, about once every month, about once every 3-6 months, or longer.

65. The combination therapy according to any one of claims 42-64, wherein the second antibody is administered intravenously.

66. The combination therapy according to any one of claims 47-65, wherein oxaliplatin is administered at a body surface area-based dose of about 10 to about 150 mg/m², about 20 to about 150 mg/m², about 30 to about 150 mg/m², about 40 to about 150 mg/m², about 50 to about 150 mg/m², about 60 to about 150 mg/m², about 70 to about 150 mg/m², about 75 to about 150 mg/m², about 80 to about 150 mg/m², about 85 to about 150 mg/m², about 90 to about 150 mg/m², about 95 to about 150 mg/m², about 100 to about 150 mg/m², about 105 to about 150 mg/m², about 110 to about 150 mg/m², about 115 to about 150 mg/m², about 120 to about 150 mg/m², about 125 to about 150 mg/m², about 130 to about 150 mg/m², about 135 to about 150 mg/m², about 140 to about 150 mg/m², about 145 to about 150 mg/m², about 10 to about 140 mg/m², about 20 to about 140 mg/m², about 30 to about 140 mg/m², about 40 to about 140 mg/m², about 50 to about 140 mg/m², about 60 to about 140 mg/m², about 70 to about 140 mg/m², about 75 to about 140 mg/m², about 80 to about 140 mg/m², about 85 to about 140 mg/m², about 90 to about 140 mg/m², about 95 to about 140 mg/m², about 100 to about 140 mg/m², about 105 to about 140 mg/m², about 110 to about 140 mg/m², about 115 to about 140 mg/m², about 120 to about 140 mg/m², about 125 to about 140 mg/m², about 130 to about 140 mg/m², about 135 to about 140 mg/m², about 10 to about 130 mg/m², about 20 to about 130 mg/m², about 30 to about 130 mg/m², about 40 to about 130 mg/m², about 50 to about 130 mg/m², about 60 to about 130 mg/m², about 70 to about 130 mg/m², about 75 to about 130 mg/m², about 80 to about 130 mg/m², about 85 to about 130 mg/m², about 90 to about 130 mg/m², about 95 to about 130 mg/m², about 100 to about 130 mg/m², about 105 to about 130 mg/m², about 110 to about 130 mg/m², about 115 to about 130 mg/m², about 120 to about 130 mg/m², or about 125 to about 130 mg/m².

67. The combination therapy according to any one of claims 47-66, wherein oxaliplatin is administered at a body surface area-based dose of about 10 mg/m², about 20 mg/m², about 30 mg/m², about 40 mg/m², about 50 mg/m², about 60 mg/m², about 70 mg/m², about 75 mg/m², about 80 mg/m², about 85 mg/m², about 90 mg/m², about 95 mg/m², about 100 mg/m², about 105 mg/m², about 110 mg/m², about 115 mg/m², about 120 mg/m², about 125 mg/m², about 130 mg/m², about 135 mg/m², about 140 mg/m², about 145 mg/m², or about 150 mg/m².

68. The combination therapy according to any one of claims 47-67, wherein oxaliplatin is administered at a body surface area-based dose of about 130 mg/m².

69. The combination therapy according to any one of claims 47-68, wherein oxaliplatin is administered about once every week, about once every two weeks, about once every three weeks, about once every four weeks, about once every month, about once every 3-6 months, or longer.

70. The combination therapy according to any one of claims 47-69, wherein oxaliplatin is administered intravenously.

71. The combination therapy according to any one of claims 48-70, wherein capecitabine is administered at a body surface area-based dose of about 100 to about 1200 mg/m², about 200 to about 1200 mg/m², about 300 to about 1200 mg/m², about 400 to about 1200 mg/m², about 500 to about 1200 mg/m², about 600 to about 1200 mg/m², about 650 to about 1200 mg/m², about 700 to about 1200 mg/m², about 750 to about 1200 mg/m², about 800 to about 1200 mg/m², about 850 to about 1200 mg/m², about 900 to about 1200 mg/m², about 950 to about 1200 mg/m², about 1000 to about 1200 mg/m², about 1050 to about 1200 mg/m², about 1100 to about 1200 mg/m², about 1150 to about 1200 mg/m², about 100 to about 1100 mg/m², about 200 to about 1100 mg/m², about 300 to about 1100 mg/m², about 400 to about 1100 mg/m², about 500 to about 1100 mg/m², about 600 to about 1100 mg/m², about 650 to about 1100 mg/m², about 700 to about 1100 mg/m², about 750 to about 1100 mg/m², about 800 to about 1100 mg/m², about 850 to about 1100 mg/m², about 900 to about 1100 mg/m², about 950 to about 1100 mg/m², about 1000 to about 1100 mg/m², about 100 to about 1000 mg/m², about 200 to about 1000 mg/m², about 300 to about 1000 mg/m², about 400 to about 1000 mg/m², about 500 to about 1000 mg/m², about 600 to about 1000 mg/m², about 650 to about 1000 mg/m², about 700 to about 1000 mg/m², about 750 to about 1000 mg/m², about 800 to about 1000 mg/m², about 850 to about 1000 mg/m², about 900 to about 1000 mg/m², or about 950 to about 1000 mg/m².

72. The combination therapy according to any one of claims 48-71, wherein capecitabine is administered at a body surface area-based dose of about 100 mg/m², about 200 mg/m², about 300 mg/m², about 400 mg/m², about 500 mg/m², about 600 mg/m², about 650 mg/m², about 700 mg/m², about 750 mg/m², about 800 mg/m², about 850 mg/m², about 900 mg/m², about 950 mg/m², about 1000 mg/m², about 1050 mg/m², about 1100 mg/m², about 1150 mg/m², or about 1200 mg/m².

73. The combination therapy according to any one of claims 48-72, wherein capecitabine is administered at a body surface area-based dose of about 1000 mg/m².

74. The combination therapy according to any one of claims 48-73, wherein capecitabine is administered once daily, twice daily, or thrice daily.

75. The combination therapy according to any one of claims 48-74, wherein capecitabine is administered orally.

76. The combination therapy according to any one of claims 42-75, wherein on a body weight basis, trastuzumab is administered at an initial loading dose of about 8 mg/kg followed by a dose of about 6 mg/kg; the second antibody is administered at a body weight-based dose of about 25 mg/kg or 15 mg/kg; oxaliplatin is administered at a body surface area-based dose of about 130 mg/m²; capecitabine is administered at a body surface area-based dose of about 1000 mg/m².

77. The combination therapy according to any one of claims 42-76, wherein the second antibody is administered to the patient before the administration of trastuzumab.

78. The combination therapy according to any one of claims 42-77, wherein the second antibody is administered to the patient after the administration of trastuzumab.

79. The combination therapy according to any one of claims 42-78, wherein trastuzumab and the second antibody are administered concurrently.

80. The combination therapy according to any one of claims 42-79, further comprising administering an immune checkpoint inhibitor.

81. The combination therapy according to claim 80, wherein the immune checkpoint inhibitor is an anti-PD-1 antibody or an anti-PD-L1 antibody.

82. The combination therapy according to claim 80 or 81, wherein the immune checkpoint inhibitor is an anti-PD-1 antibody.

83. A combination product, comprising:
trastuzumab;
a second antibody, wherein the second antibody is a second anti-HER2 antibody or an antigen-binding fragment thereof having a different binding site from trastuzumab, comprising:
a heavy chain variable region, comprising the HCDR1 set forth in SEQ ID NO: 1, the HCDR2 set forth in SEQ ID NO: 2, and the HCDR3 set forth in SEQ ID NO: 3, and
a light chain variable region, comprising the LCDR1 set forth in SEQ ID NO: 6, the LCDR2 set forth in SEQ ID NO: 7, and the LCDR3 set forth in SEQ ID NO: 8; and
a chemotherapeutic agent.

84. The combination product according to claim 83, wherein the chemotherapeutic agent is a fluorouracil drug.

85. The combination product according to claim 83, wherein the chemotherapeutic agent is a platinum-based drug.

86. The combination product according to any one of claims 83-85, wherein the chemotherapeutic agent is a combination of a fluorouracil drug and a platinum-based drug.

87. The combination product according to any one of claims 84-86, wherein the fluorouracil drug is selected from 5-fluorouracil and capecitabine.

88. The combination product according to any one of claims 85-87, wherein the platinum-based drug is selected from cisplatin and oxaliplatin.

89. The combination product according to any one of claims 84-88, wherein the fluorouracil drug is capecitabine.

90. The combination product according to any one of claims 85-89, wherein the platinum-based drug is oxaliplatin.

91. The combination product according to any one of claims 83-90, wherein the chemotherapeutic agent is a combination of capecitabine and oxaliplatin.

92. The combination product according to any one of claims 83-91, wherein the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

93. The combination product according to any one of claims 83-92, wherein the heavy chain of the second antibody comprises or consists of the amino acid sequence set forth in SEQ ID NO: 5, and the light chain of the second antibody comprises or consists of the amino acid sequence set forth in SEQ ID NO: 10.

94. The combination product according to any one of claims 83-93, further comprising an anti-PD-1 antibody and/or an anti-PD-L1 antibody.

95. The combination product according to any one of claims 83-94, further comprising an anti-PD-1 antibody.

96. A kit for use in treating HER2-positive gastric cancer or gastroesophageal junction adenocarcinoma, comprising:
trastuzumab;
a second antibody, wherein the second antibody is a second anti-HER2 antibody or an antigen-binding fragment thereof having a different binding site from trastuzumab, comprising:
a heavy chain variable region, comprising the HCDR1 set forth in SEQ ID NO: 1, the HCDR2 set forth in SEQ ID NO: 2, and the HCDR3 set forth in SEQ ID NO: 3, and
a light chain variable region, comprising the LCDR1 set forth in SEQ ID NO: 6, the LCDR2 set forth in SEQ ID NO: 7, and the LCDR3 set forth in SEQ ID NO: 8; and
a chemotherapeutic agent.

97. The kit according to claim 96, wherein the chemotherapeutic agent is a fluorouracil drug.

98. The kit according to claim 96, wherein the chemotherapeutic agent is a platinum-based drug.

99. The kit according to any one of claims 96-98, wherein the chemotherapeutic agent is a combination of a fluorouracil drug and a platinum-based drug.

100. The kit according to any one of claims 97-99, wherein the fluorouracil drug is selected from 5-fluorouracil and capecitabine.

101. The kit according to any one of claims 98-100, wherein the platinum-based drug is selected from cisplatin and oxaliplatin.

102. The kit according to any one of claims 97-101, wherein the fluorouracil drug is capecitabine.

103. The kit according to any one of claims 98-102, wherein the platinum-based drug is oxaliplatin.

104. The kit according to any one of claims 96-103, wherein the chemotherapeutic agent is a combination of capecitabine and oxaliplatin.

105. The kit according to any one of claims 96-104, wherein the cancer is HER2-positive advanced gastric cancer.

106. The kit according to any one of claims 96-105, wherein the cancer is unresectable or metastatic HER2-positive gastric cancer or gastroesophageal junction adenocarcinoma.

107. The kit according to any one of claims 96-106, wherein the heavy chain variable region of the second antibody comprises or consists of the amino acid sequence set forth in SEQ ID NO: 4, and the light chain variable region of the second antibody comprises or consists of the amino acid sequence set forth in SEQ ID NO: 9.

108. The kit according to any one of claims 96-107, wherein the heavy chain of the second antibody comprises or consists of the amino acid sequence set forth in SEQ ID NO: 5, and the light chain of the second antibody comprises or consists of the amino acid sequence set forth in SEQ ID NO: 10.

109. The kit according to any one of claims 96-108, further comprising a package insert.

110. The kit according to any one of claims 96-109, further comprising an anti-PD-1/PD-L1 antibody.

111. The kit according to any one of claims 96-110, further comprising an anti-PD-1 antibody.
